# EUROPÄISCHE PATENTSCHRIFT

(11) **EP 2 135 587 B1**
(45) Veröffentlichungstag und Bekanntmachung des Hinweises auf die Patenterteilung: **18.03.2015**
(21) Anmeldenummer: 09163069.9
(22) Anmeldetag: 18.06.2009
(51) Int. Cl.: A61F 2/30, A61F 2/34, A61F 2/36, A61F 2/46

(54) **Modulares Probeimplantatsystem**
Modular sample implant system
Système d'implant d'échantillon modulaire

(30) Priorität: 18.06.2008 DE 102008030261
(43) Veröffentlichungstag der Anmeldung: 23.12.2009
(73) Patentinhaber: Aesculap AG, 78532 Tuttlingen (DE)
(72) Erfinder: Reu, Gerhard, 78532, Tuttlingen (DE); Fritzsche, Jörg, 78665, Frittlingen (DE); Hermle, Thomas, 78628, Rottweil (DE)
(74) Vertreter: Hoeger, Stellrecht & Partner Patentanwälte

(56) Entgegenhaltungen:
- US-A1- 2004 117 029
- US-A1- 2006 217 815
- US-A1- 2008 109 001

## Beschreibung

Die vorliegende Erfindung betrifft ein Probeimplantatsystem umfassend mindestens ein Probeimplantat, welches mindestens einen ersten und mindestens einen zweiten, mit dem mindestens einen ersten Probeimplantatteil verbindbaren Probeimplantatteil umfasst, wobei mindestens eine Verbindungseinrichtung zum Verbinden des mindestens einen ersten und des mindestens einen zweiten Probeimplantatteils in einer Probeimplantationsstellung vorgesehen ist, wobei die mindestens eine Verbindungseinrichtung mindestens ein erstes und mindestens ein zweites Verbindungselement umfasst, wobei das eine Verbindungselement am mindestens einen ersten Probeimplantatteil und wobei das andere Verbindungselement am mindestens einen zweiten Probeimplantatteil angeordnet ist und wobei das mindestens eine erste und das mindestens eine zweite Verbindungselement korrespondierend zueinander ausgebildet sind und in der Probeimplantationsstellung in Eingriff stehen.

In der Chirurgie, insbesondere in der Hüftendoprothetik, werden intraoperativ Proberepositionen durchgeführt, um einen Bewegungsbereich, den sogenannten "Range of Motion" (ROM), die Bandspannung und gegebenenfalls eine Beinlängenveränderung zu prüfen. Es ist bekannt, Probeimplantate in Form von Gelenkpfannen sowie in Form von Gelenkschäften einzusetzen. Um insbesondere den physiologischen Anforderungen jedes Patienten optimal gerecht werden und auch auf die neuestens Erkenntnisse der Tribologie reagieren zu können, stieg in den letzten Jahren die Tendenz, immer größere Durchmesser der Artikulationspartner, also zum Beispiel Gelenkpfanne und Gelenkkopf bei einem künstlichen Hüftgelenk, einzusetzen. Die Folge hiervon ist, dass die Anzahl der für eine Operation bereitzuhaltenden Probeimplantate kontinuierlich zunahm und noch weiter zunimmt. Dies bedeutet zum einen, dass diese Probeimplantate bereitgehalten werden müssen, und zum anderen, dass auch durch die größere Anzahl an Probeimplantaten ein größerer Reinigungs- und Wiederaufbereitungsaufwand betrieben werden muss. Beispielsweise sind bei einem Probeimplantatsystem umfassend Gelenkköpfe mit fünf unterschiedlichen Halslängen (S, M, L, XL, XXL) und drei unterschiedlichen Durchmessern, (zum Beispiel 28 mm, 32 mm und 36 mm), insgesamt 15 verschiedene Probeköpfe bereitzustellen. Entsprechendes gilt auch für die Probegelenkpfannen sowie lateralisierte Varianten von Probegelenkköpfen. Insgesamt wären also eine zunehmend unüberschaubare Zahl von Probeimplantaten erforderlich, um vor der Implantation der eigentlichen Implantats, Größe und Position desselben optimal ermitteln zu können.

Aus der US 2008/0109001 A1 ist ein Probeimplantatsystem betreffend einen Hüftgelenkendoprothesenkopf bekannt. In der US 2004/0117029 A1 ist ein Probeimplantatsystem betreffend eine Hüftgelenkendoprothesenpfanne offenbart. Die US 2006/0217815 A 1 beschreibt einen modularen Prothesenkopf einer Hüftgelenkendoprothese.

Es ist daher Aufgabe der vorliegenden Erfindung, ein Probeimplantatsystem der eingangs beschriebenen Art so zu verbessern, dass es einen besonders einfachen Aufbau aufweist.

Diese Aufgabe wird bei einem Probeimplantatsystem der eingangs beschriebenen Art erfindungsgemäß dadurch gelöst, dass die mindestens eine Verbindungseinrichtung in Form einer Rastverbindung ausgebildet ist.

Die erfindungsgemäß vorgeschlagene Lösung ermöglicht es, insbesondere Probeimplantatparameter zu separieren, indem beispielsweise ein erster Probeimplantatparameter dem mindestens einen ersten Probeimplantatteil zugeordnet und ein zweiter Probeimplantatparameter dem mindestens einen zweiten Probeimplantatteil zugeordnet wird. Dadurch, dass die mindestens einen ersten und die mindestens einen zweiten Probeimplantatteile vorzugsweise so ausgebildet sind, dass die ersten und zweiten Probeimplantatteile frei miteinander kombiniert werden können, kann so die Zahl der insgesamt erforderlichen Probeimplantatteile deutlich reduziert werden. Bezogen auf das eingangs genannte Beispiel bedeutet dies, dass beispielsweise fünf erste Probeimplantatteile genügen, um die fünf unterschiedlichen Halslängen abzubilden. Falls die fünf ersten Probeimplantatteile zusätzlich alle denselben Kopfdurchmesser aufweisen, reichen zusätzlich zwei mit den fünf ersten Probeimplantatteilen verbindbare zweite Implantatteile mit unterschiedlichen Kopfdurchmessern aus, um insgesamt die erforderlichen fünfzehn Varianten auszubilden. Somit sind in diesem Fall nur fünf erste und zwei zweite, also insgesamt nur sieben Probeimplantatteile erforderlich. Die Erfindung ermöglicht es also, die Zahl der Probeimplantate deutlich zu verringern, im Falle des betrachteten Beispiels sogar auf weniger als die Hälfte. Insgesamt lassen sich so die Kosten für die Bereitstellung des Probeimplantatsystems senken, ebenso Aufwand und Kosten für die Reinigung. Des Weiteren kann ein solches Probeimplantatsystem zukünftig ohne Weiteres durch entsprechende weitere Implantatteile ergänzt werden, beispielsweise um Probeimplantatparameter noch feiner abzustufen oder um überhaupt weitere Probeimplantatteilparameter zu berücksichtigen. Eine Verbindung der mindestens einen ersten und mindestens einen zweiten Probeimplantatteile miteinander wird besonders einfach dadurch, dass mindestens eine Verbindungseinrichtung zum Verbinden des mindestens einen ersten und des mindestens einen zweiten Probeimplantatteils in einer Probeimplatationsstellung vorgesehen ist. Der Aufbau der mindestens einen Verbindungseinrichtung wird besonders einfach dadurch, dass die mindestens eine Verbindungseinrichtung mindestens ein erstes und mindestens ein zweites Verbindungselement umfasst, wenn das eine Verbindungselement am mindestens einen ersten Probeimplantatteil und wenn das andere Verbindungselement am mindestens einen zweiten Probeimplantatteil angeordnet ist und wenn das mindestens eine erste und das mindestens eine zweite Verbindungselement korrespondierend zueinander ausgebildet sind und in der Implantationsstellung in Eingriff stehen. Die ersten und zweiten Verbindungselemente können grundsätzlich beliebig ausgebildet sind. Vorzugsweise sind sie so ausgebildet, dass eine Trennung der Probeimplantatteile sicher verhindert wird, wenn nicht mindestens eine minimale Lösekraft aufgebracht oder ein entsprechendes Löseinstrument eingesetzt wird, um die Probeimplantatteile voneinander zu lösen. Ein besonders einfacher Aufbau der Verbindungseinrichtung wird dadurch erreicht, dass sie in Form einer Rastverbindung ausgebildet ist. Mit anderen Worten bedeutet dies, dass die ersten und zweiten Probeimplantatteile, die miteinander zusammen verbunden sind und ein Probeimplantat oder einen Teil desselben ausbilden, kraft- und/oder formschlüssig miteinander verbunden sein können. Alternativ oder zusätzlich können auch sie durch entsprechende Rast- oder Schraubverbindungen aneinander gesichert beziehungsweise miteinander verbunden sein.

Damit die einzelnen Probeimplantatteile optimal gereinigt werden können, ist es günstig, wenn der mindestens eine erste und der mindestens eine zweite Probeimplantatteil miteinander lösbar verbindbar sind. So kann zudem intraoperativ eine Anpassung durch Voneinanderlösen von Probeimplantatteilen und neues Verbinden anderer Probeimplantatteile miteinander erreicht werden.

Insbesondere ohne zusätzliche Instrumente oder Werkzeuge können die ersten und zweiten Probeimplantatteile miteinander verbunden werden, wenn das mindestens eine erste und das mindestens eine zweite Verbindungselement in Form zueinander korrespondierender Rastelemente ausgebildet sind. Vorzugsweise ist mindestens eines der Rastelemente relativ zum anderen Rastelement, mit welchem es in Eingriff gebracht werden soll, beweglich an einem der Probeimplantatteile gehalten, um so ein einfaches Einschnappen oder Verrasten beim Zusammenbringen der miteinander zu verbindenden Probeimplantatteile zu ermöglichen. Günstigerweise ist eines der Rastelemente in Form eines oder mehrerer Spannfinger ausgebildet und das korrespondierende Rastelement in Form eines Hinterschnitts.

Die Herstellung und Konstruktion der Rastelemente wird besonders einfach, wenn das eine Rastelement in Form einer Ringnut oder eines Ringnutabschnitts oder wenn das korrespondierende andere Rastelement in Form eines Ringwulstes oder eines Ringwulstabschnitts ausgebildet ist, welcher Ringwulst oder Ringwulstabschnitte in der Probeimplantationsstellung in die Ringnut oder den Ringnutabschnitt eintaucht oder eingreift. Insbesondere kann ein Ringwulst oder Ringwulstabschnitt auch ein Rastelement in Form einer Rastkante mindestens teilweise hintergreifen, die dann im Sinne einer zeitlich geöffneten Ringnut oder einer seitlich geöffneten Ringnutabschnitts ausgebildet ist.

Auf einfache Weise lassen sich die ersten und zweiten Probeimplantatteile auch miteinander verbinden, wenn das mindestens eine erste und/oder das mindestens eine zweite Verbindungselement in Form von korrespondierenden Aufnahmen und Vorsprüngen ausgebildet sind, die in der Probeimplantationsstellung kraft- und/oder formschlüssig miteinander verbunden sind. Denkbar sind hier insbesondere zapfenförmige Vorsprünge und korrespondierende Aufnahmen, beispielsweise in Form von Sacklöchern oder Bohrungen, die ein Ineingriffbringen der Verbindungselemente auf einfache Weise ermöglichen. Zusätzlich können noch weitere Rast- oder Verbindungselemente vorgesehen sein, um eine Verbindung zwischen den Probeimplantatteilen zu stabilisieren. Insbesondere können die Aufnahmen und Vorsprünge auch mit Gewindeabschnitten versehen sein, um so ein Verschrauben der Probeimplantatteile miteinander zu ermöglichen.

Eine optimale Sicherung der miteinander zu verbindenden Probeimplantatteile in der Probeimplantationsstellung kann dadurch erreicht werden, dass das mindestens eine erste und das mindestens eine zweite Rastelement an den korrespondierenden Aufnahmen und Vorsprüngen angeordnet sind. So werden ferner auch keine weiteren zusätzlichen Teile oder Elemente benötigt, um die Probeimplantatteile miteinander zu verbinden.

Günstig ist es, wenn das eine der mindestens zwei Verbindungselemente einen Innengewindeabschnitt und das andere der mindestens zwei Verbindungselemente einen korrespondierenden Außengewindeabschnitt aufweisen. So können die Verbindungselemente miteinander verschraubt und die Probeimplantatteile dadurch miteinander verbunden werden.

Um einer Bedienperson auf einfache Weise das Verbinden und Voneinanderlösen der Probeimplantatteile zu gestatten, ist es vorteilhaft, wenn die Verbindungseinrichtung rotationssymmetrisch bezogen auf eine Probeimplantat-längsachse ausgebildet ist. Ferner kann so auch die Herstellung der Probeimplantatteile vereinfacht werden.

Die Zahl der erforderlichen Probeimplantatteile lässt sich weiter verringern, wenn der mindestens eine erste Probeimplantatteil einen Grundkörper des Probeimplantatsystems bildet. Insbesondere kann der Grundkörper derart ausgebildet sein, dass er zwei Probeimplantatparameter definiert, beispielsweise einen Kopfdurchmesser und ein Halslänge. Durch entsprechende Kombination mit einem zweiten Probeimplantatteil, welches einen der beiden Probeimplantatparameter ändert, wenn die Probeimplantatteile miteinander verbunden sind, kann so ein Probeimplantatsystem mit einer minimalen Anzahl von Probeimplantatteilen oder Komponenten bereitgestellt werden.

Die Stabilität des Probeimplantats kann auf einfache Weise dadurch verbessert werden, wenn das mindestens eine erste Probeimplantatteil einstückig ausgebildet ist.

Vorteilhafterweise ist das mindestens eine zweite Probeimplantatteil einstückig ausgebildet. Dies erhöht insbesondere die Stabilität des Probeimplantatsystems. Ferner kann so auch die Herstellung und Konstruktion der Probeimplantatteile weiter vereinfacht werden.

Günstigerweise sind mindestens zwei unterschiedlich geformte erste Probeimplantatteile vorgesehen. Insbesondere sind sie derart unterschiedlich geformt, dass sie einen ersten Probeimplantatparameter, beispielsweise eine Halslänge oder einen Kopfdurchmesser, definieren, die bei den unterschiedlich geformten ersten Probeimplantatteilen unterschiedlich sind. Werden beispielsweise die mindestens zwei unterschiedlich geformten ersten Probeimplantatteile mit demselben zweiten Probeimplantatteil verbunden, so ergeben sich entsprechende Kombinationsmöglichkeiten der Probeimplantatteile miteinander und eine verringerte Zahl an für das Probeimplantatsystem insgesamt erforderlichen Komponenten.

Vorzugsweise sind mindestens zwei unterschiedlich geformte zweite Probeimplantatteile vorgesehen. Diese gestatten es, insbesondere dann, wenn sie einen Probeimplantatparameter durch ihre unterschiedliche Form variieren, durch entsprechende Verbindung mit einem oder mehreren ersten Probeimplantatteilen eine Mehrzahl unterschiedlicher Probeimplantate insgesamt auszubilden.

Vorteilhafterweise definiert der mindestens eine erste Probeimplantatteil mindestens zwei Probeimplantatteilparameter. Insbesondere kann es sich bei den mindestens zwei Probeimplantatteilparametern um Probeimplantatparameter handeln. Beispielsweise kann es sich bei einem Probeimplantatparameter eines Probeimplantats zur Verwendung im Zusammenhang mit der Implantation eines künstlichen Hüftgelenks um eine Halslänge oder einen Gelenkkopfdurchmesser handeln. Vorzugsweise ist diese Ausgestaltung bei einem Grundkörper des Probeimplantats realisiert. Dies ermöglicht es, durch Verbinden des ersten Probeimplantatteils mit einem zweiten Probeimplantatteil, einen der beiden Probeimplantatteilparameter zu variieren. Es ist insbesondere zur Minimierung der Gesamtzahl der Komponenten des Probeimplantatsystems günstig, wenn alle ersten Probeimplantatteile, die mindestens zwei Probeimplantatteilparameter definieren, jeweils einen identischen Wert für denselben Probeimplantatteilparameter aufweisen. Dabei kann es sich beispielsweise um eine Halslänge oder um einen Gelenkkopfdurchmesser handeln.

Günstig ist es, wenn sich die mindestens zwei unterschiedlich geformten ersten Probeimplantatteile in mindestens einem ersten Probeimplantatteilparameter unterscheiden. So kann insbesondere dann, wenn ein weiterer Probeimplantatteilparameter bei den unterschiedlich geformten ersten Probeimplantatteilen identisch ist, beispielsweise durch ein einziges weiteres zweites Probeimplantatteil, das mit den mindestens zwei unterschiedlich geformten ersten Probeimplantatteilen verbindbar ist, jeweils ein anderes Probeimplantat ausgebildet werden. Zur Verdopplung der Zahl der aus den Probeimplantatteilen kombinierbaren Probeimplantaten ist in einem solchen Fall nur ein einziges weiteres zweites Probeimplantatteil erforderlich.

Vorzugsweise sind zwei, drei, vier, fünf, sechs oder mehr erste Probeimplantatteile vorgesehen.

Günstigerweise sind zwei, drei, vier, fünf, sechs oder mehr mit dem mindestens einen ersten Probeimplantatteil verbindbare zweite Probeimplantatteile vorgesehen. Insbesondere ist es damit möglich, mit N₁ ersten Probeimplantatteilen und N₂ zweiten Probeimplantatteilen insgesamt N=N₁xN₂ Probeimplantate auszubilden. Hierfür sind dann nur N_{G} = N₁+N₂ Probeimplantatteile oder Komponenten erforderlich.

Ferner kann es vorteilhaft sein, wenn der mindestens eine zweite Probeimplantatteil mindestens zwei Probeimplantatteilparameter definiert. Auf diese Weise kann die Zahl der erforderlichen Komponenten des Probeimplantatsystems weiter verringert werden.

Günstigerweise unterscheiden sich die mindestens zwei unterschiedlich geformten zweiten Probeimplantatteile in mindestens einem zweiten Probeimplantatteilparameter. Dadurch können durch Kombination mit den mindestens zwei unterschiedlich geformten zweiten Probeimplantatteilen mit einem einzigen weiteren ersten Probeimplantatteil mindestens zwei Probeimplantate ausgebildet werden.

Günstigerweise bildet das mindestens eine Probeimplantat mindestens einen Teil eines ersten Probegelenkteils eines künstlichen Probegelenks. Es kann auch insgesamt einen ersten Probegelenkteil des künstlichen Probegelenks bilden. Das Probeimplantat selbst umfasst mindestens ein erstes und mindestens ein zweites Probeimplantatteil.

Damit die Probeimplantate in Verbindung mit einem chirurgischen Eingriff zum Implantieren eines künstlichen Hüftgelenks eingesetzt werden können, ist es vorteilhaft, wenn das künstliche Probegelenk ein künstliches Probehüftgelenk mit einer Probegelenkpfanne und einem Probegelenkkopf ist.

Günstigerweise umfasst das Probeimplantatsystem mindestens ein Probeimplantat in Form eines Probegelenkkopfs. Dieser kann einstückig oder lösbar verbindbar mit einem Probeschaft ausgebildet sein. Optional kann der Probegelenkkopf auch einen Halsteil umfassen.

Um die Zahl der Komponenten des Probeimplantatsystems weiter zu verringern, ist es günstig, wenn der Probegelenkkopf einen Gelenkkopfteil und einen Verbindungsteil umfasst. Insbesondere können sowohl der Gelenkkopfteil als auch der Verbindungsteil jeweils mindestens einen Implantatteilparameter definieren, beispielsweise der Gelenkkopfteil einen Gelenkkopfdurchmesser und der Verbindungsteil eine Halslänge. Dies gestattet es, aus einer Gesamtzahl von Probeimplantatteilen, die der Summe der Anzahl der bereitgestellten Gelenkkopfteile und Verbindungsteile entspricht, insgesamt so viele Probegelenkköpfe auszubilden, wie dem Produkt der Zahl der Gelenkkopfteile und der Verbindungsteile entspricht.

Vorzugsweise umfasst das mindestens eine erste Probeimplantatteil den Gelenkkopfteil und den Verbindungsteil. Es kann insbesondere einstückig ausgebildet sein und so gleichzeitig zwei Probeimplantatteilparameter definieren, und zwar beispielsweise einen, der eine Eigenschaft des Gelenkkopfteils beschreibt oder definiert, und einen, welcher eine Eigenschaft des Verbindungsteils beschreibt oder definiert.

Gemäß einer bevorzugten Ausführungsform der Erfindung kann vorgesehen sein, dass der Verbindungsteil eine Halsaufnahme umfasst, in welche ein zapfenförmiger Halsabschnitt eines Gelenkschafts einführbar ist zum Verbinden des Probegelenkkopfs mit dem Gelenkschaft. Bei dem Gelenkschaft kann es sich insbesondere um einen Probegelenkschaft oder um einen bereits in einen Knochen des Patienten implantierten Gelenkschaft der zu implantierenden Prothese handeln. Durch diese besondere Ausgestaltung des Verbindungsteils ist es möglich, noch nach Einsetzen des zu implantierenden Prothesenschafts das optimale, der jeweiligen Physiologie entsprechende Gelenkkopfteil der Gelenkprothese zu ermitteln, und zwar durch Ausprobieren unterschiedlicher Probegelenkköpfe, welche mit dem Gelenkschaft verbunden werden.

Vorteilhafterweise ist der mindestens eine zweite Probeimplantatteil in Form einer Gelenkkopfschale ausgebildet. Mit der Gelenkkopfschale kann auf einfache Weise durch Verbinden mit einem Gelenkkopf des ersten Implantatteils dessen Durchmesser vergrößert werden. Durch Verbinden der ersten und zweiten Probeimplantatteile kann so auf einfache Weise ein weiteres, modular aufgebautes Probeimplantat ausgebildet werden.

Insbesondere ist es günstig, wenn die Gelenkkopfschale mit dem Gelenkkopfteil verbindbar ist. Dies ermöglicht es, nur jeweils durch Anbringen einer Gelenkkopfschale am Gelenkkopfteil dessen Durchmesser zu verändern. Optional können auch Gelenkkopfschalen unterschiedlicher Größe so ausgebildet sein, dass sie miteinander und mit einem Gelenkkopfteil verbunden werden können. Auf diese Weise lässt sich die Zahl der insgesamt vom Probeimplantatsystem umfassten Komponenten weiter verringern.

Vorzugsweise definiert der Probegelenkkopf mindestens zwei Probeimplantatteilparameter. Der Probegelenkkopf kann dann beispielsweise durch Verbinden mit weiteren Probeimplantatteilen variiert werden, wobei je nach Ausgestaltung des Probeimplantatteils ein, zwei oder auch mehr Probeimplantatteilparameter gleichzeitig variiert werden können.

Besonders einfach und effizient wird der Aufbau des Probeimplantatsystems, wenn der mindestens eine vom Probegelenkkopf definierte erste Probeimplantatteilparameter einen Gelenkkopfdurchmesser des Probeimplantats definiert. So kann durch einfache Verbindung mit weiteren Probeimplantatteilen der Gelenkkopfdurchmesser variiert werden, beispielsweise durch Aufbringen oder Aufschnappen von Gelenkkopfschalen auf den Probegelenkkopf.

Vorteilhafterweise definiert der mindestens eine vom Probegelenkkopf definierte zweite Probeimplantatteilparameter eine Verbindungsteillänge des Probeimplantats. Die Verbindungsteillänge kann insbesondere auch eine Halslänge des Probegelenkkopfs definieren. Optional kann mit Probeimplantatteilen, die mit dem Verbindungsteil verbindbar sind, durch entsprechende Wahl unterschiedlicher Längen der Verbindungsteile eine Verbindungsteillänge des Probeimplantats variiert werden.

Gemäß einer bevorzugten Ausführungsform der Erfindung kann ferner vorgesehen sein, dass der mindestens eine erste Probeimplantatteil einen ersten Probeimplantatteilparameter definiert und dass der mit ihm verbindbare mindestens eine zweite Probeimplantatteil einen größeren ersten Probeimplantatteilparameter definiert. Beispielsweise kann so durch Verbinden des ersten und zweiten Probeimplantatteils miteinander ein Gelenkkopfdurchmesser vergrößert werden, wenn nämlich der einen Probeimplantatteilparameter bildende Gelenkkopfdurchmesser des zweiten Probeimplantatteils größer ist als der des ersten Probeimplantatteils, mit welchem der zweite Probeimplantatteil verbunden wird.

Alternativ kann es auch vorteilhaft sein, wenn der mindestens eine erste Probeimplantatteil einen ersten Probeimplantatteilparameter definiert und wenn der mit ihm verbindbare eine zweite Probeimplantatteil einen kleineren ersten Probeimplantatteilparameter definiert. Beispielsweise kann diese Ausgestaltung vorteilhaft sein bei Probegelenkpfannen, wobei sich Innendurchmesser von Pfanneneinsätzen, welche erste Probeimplantatteile bilden, durch Verbinden mit zweiten Probeimplantatteilen, sogenannten Reduktionsteilen, verringern lassen, beispielsweise zum Anpassen der Probegelenkpfanne mit ihrem Pfanneneinsatz an einen im Durchmesser kleineren Gelenkkopf.

Vorzugsweise definiert der erste Probeimplantatteilparameter eine minimale Verbindungsteillänge. Die Verbindungsteillänge kann dann auf einfache Weise zum Beispiel durch Verbinden mit weiteren Verbindungsteilen in gewünschter Weise verlängert werden.

Um durch Verbinden mit einem zweiten Probeimplantatteil einen Gelenkkopfdurchmesser des Probeimplantats auf einfache Weise vergrößern zu können, ist es günstig, wenn der mindestens eine erste Probeimplantatteil einen minimalen Gelenkkopfdurchmesser definiert.

Günstigerweise ist der mindestens eine erste Probeimplantatteil mit mindestens zwei zweiten Probeimplantatteilen verbindbar. Er kann selbstverständlich auch mit drei, vier, fünf, sechs oder mehr zweiten Implantatteilen verbindbar sein. So kann auf einfache Weise durch Erhöhung der Zahl der Komponenten des Probeimplantatsystems um eine weitere Komponente die Zahl der insgesamt ausbildbaren Probeimplantate vervielfacht werden.

So kann die Zahl der Probeimplantate insbesondere auf einfache Weise durch nur eine weitere Komponente vervielfacht werden, wenn der mindestens eine zweite Probeimplantatteil mit mindestens zwei ersten Probeimplantatteilen verbindbar ist. Insgesamt können so beliebige Permutationen der bereitgestellten Probeimplantatteile durch Verbinden derselben erreicht werden.

Vorzugsweise umfasst das Probeimplantatsystem ein Probeimplantat in Form eines mit einer Probegelenkpfanne oder Gelenkpfanne verbindbaren Pfanneneinsatzes, welcher mit einem korrespondierenden Probegelenkkopf in Eingriff bringbar ist. So kann zu einem Gelenkkopf auch die passende Gelenkpfanne mit zugehörigem Pfanneneinsatz durch Probieren ermittelt werden. Nach Bestimmung der geeigneten Größe des Pfanneneinsatzes kann dann der entsprechende Pfanneneinsatz zur Ausbildung des künstlichen Gelenks mit der Gelenkpfanne nach Implantieren derselben verbunden werden. Die Pfanneneinsätze sind vorzugsweise so ausgebildet, dass sie mit passenden Probegelenkköpfen, dies bedeutet insbesondere Probegelenkköpfe mit entsprechenden Gelenkkopfdurchmessern, verbunden werden können, um auch eine Beweglichkeit des Gelenks zu prüfen.

Günstigerweise umfasst der Pfanneneinsatz einen Pfanneneinsatzteil und einen Pfannenverbindungsteil. Die Trennung des Pfanneneinsatzes in die beiden Teile ermöglicht es, durch Variation derselben beispielsweise nur jeweils einen Probeimplantatteilparameter zu variieren. Es ist zu beachten, dass Pfanneneinsatzteil und Pfannenverbindungsteil auch einstückig miteinander ausgebildet oder dauerhaft fest oder unlösbar miteinander verbunden sein können.

Vorteilhafterweise umfasst der mindestens eine erste Probeimplantatteil den Pfanneneinsatzteil und den Verbindungsteil. Insbesondere ist es so möglich, eine Grundtype zur Ausbildung eines Pfanneneinsatzes zu konstruieren und gegebenenfalls mit zweiten Probeimplantatteilen zu verbinden, um durch den Pfanneneinsatzteil und/oder den Pfannenverbindungsteil definierte Parameter gezielt zu variieren.

Vorzugsweise ist der mindestens eine zweite Probeimplantatteil in Form eines Einsatzverkleinerungsteils ausgebildet. Dieser ist insbesondere geeignet, Pfanneneinsätze zu verkleinern, das heißt beispielsweise einen Innendurchmesser des Pfanneneinsatzes. Derartige Einsatzverkleinerungsteile werden auch als "Durchmesserreduktionen" oder auch nur als "Reduktionen" bezeichnet.

Auf einfache Weise kann ein Innendurchmesser des Pfanneneinsatzteils verringert werden, wenn das Einsatzverkleinerungsteil mit dem Pfanneneinsatz verbindbar ist. Günstigerweise weisen dabei das Pfanneneinsatzteil und das Einsatzverkleinerungsteil unterschiedliche Werte für denselben Probeimplantatteilparameter auf.

Um eine optimale Anpassung eines künstlichen Gelenks an eine Physiologie des Patienten zu ermöglichen, ist es vorteilhaft, wenn der Pfanneneinsatz mindestens zwei Probeimplantatteilparameter definiert. So können insbesondere sukzessive, also zum Beispiel nacheinander, Probeimplantatteilparameter gezielt variiert und Probeimplantate ausgebildet werden, die an eine Physiologie des Patienten bestens angepasst sind.

Vorteilhaft ist es, wenn der mindestens eine vom Pfanneneinsatz definierte erste Probeimplantatteilparameter einen Pfanneneinsatzinnendurchmesser des Probeimplantats definiert. So kann durch Auswahl entsprechender Pfanneneinsätze und zugehöriger Einsatzverkleinerungsteile ein zum Gelenkkopf optimaler Innendurchmesser vorgegeben werden.

Um zusätzlich einen Abstand zwischen Gelenkkopf und Gelenkpfanne variieren zu können, ist es günstig, wenn der mindestens eine vom Pfanneneinsatz definierte zweite Probeimplantatteilparameter eine Pfannenhöhe des Probeimplantats definiert.

Vorteilhafterweise definiert der mindestens eine erste Probeimplantatteil eine minimale Pfannenhöhe. Die Pfannenhöhe zu erhöhen kann dann insbesondere auf einfache Weise durch Verbinden des mindestens einen ersten Probeimplantatteils mit einem zweiten Probeimplantatteil erreicht werden.

Optional kann es auch vorteilhaft sein, wenn der mindestens eine erste Probeimplantatteil eine maximale Pfannenhöhe definiert.

Auf einfache Weise können Probeimplantate ausgebildet werden mit unterschiedlichen Pfanneninnendurchmessern beziehungsweise Pfanneneinsatzinnendurchmessern, wenn der mindestens eine erste Probeimplantatteil einen maximalen Pfanneneinsatzinnendurchmesser definiert. Eine Reduktion des Pfanneneinsatzinnendurchmessers kann dann insbesondere erreicht werden durch Einsetzen eines Pfannenverkleinerungseinsatzes in den Pfanneneinsatz.

Zur Ausbildung von Probegelenken ist es günstig, wenn mindestens ein korrespondierend zum mindestens einen Probegelenkkopf ausgebildeter Pfanneneinsatz vorgesehen ist.

Des Weiteren ist es günstig, wenn das Probeimplantatsystem mindestens einen Probeschaft umfasst, mit welchem der Probegelenkkopf verbindbar ist. So kann das Probeimplantatsystem insbesondere auch genutzt werden, um einen passenden Gelenkschaft auszusuchen oder einen Probegelenkschaft auszubilden.

Ferner ist es vorteilhaft, wenn das Probeimplantatsystem mindestens eine Probegelenkpfanne umfasst, mit welcher der mindestens eine Pfanneneinsatz verbindbar ist. So kann wahlweise auch ausprobiert werden, welche Probgelenkpfanne optimal zur Physiologie des Patienten passt.

Die nachfolgende Beschreibung bevorzugter Ausführungsformen der Erfindung dient im Zusammenhang mit der Zeichnung der näheren Erläuterung. Es zeigen:
- Figur 1a:: eine perspektivische Ansicht eines ersten Ausführungsbeispiels eines Probeimplantats;
- Figur 1b:: eine teilweise geschnittene Explosionsdarstellung des Probeimplantats aus Figur 1a;
- Figur 2a:: eine Längsschnittansicht des zweiteiligen Probeimplantats aus Figur 1a;
- Figur 2b:: eine Längsschnittansicht analog Figur 2a eines Probeimplantats mit einem modifizierten ersten Implantatteil beziehungsweise einem zweiten Probeimplantatgrundkörper;
- Figur 2c:: eine Längsschnittansicht analog Figur 2a eines weiteren Probeimplantatteils des Probeimplantatsystems mit einem dritten Probeimplantatgrundkörper;
- Figur 2d:: eine Längsschnittansicht analog Figur 2a eines weiteren Probeimplantats mit einem vierten Probeimplantatgrundkörper;
- Figur 3:: eine schematische Übersicht von Längsschnittansichten von zwölf Probeimplantaten eines Probeimplantatsystems umfassend vier erste und zwei zweite Probeimplantatteile;
- Figuren 4a bis 4d:: Längsschnittansichten analog den Figuren 2a bis 2d von vier Probeimplantaten einer alternativen Ausführungsform eines modularen Probeimplantatsystems;
- Figuren 5a bis 5d:: Längsschnittansichten analog den Figuren 2a bis 2d von vier Probeimplantaten eines weiteren Ausführungsbeispiels eines modularen Probeimplantatsystems;
- Figur 6a:: eine Längsschnittansicht eines weiteren Probeimplantats eines Probeimplantatsystems;
- Figur 6b:: eine Längsschnittansicht analog Figur 6a eines weiteren Probeimplantats;
- Figur 6c:: eine Längsschnittansicht analog Figur 6a eines weiteren Probeimplantats;
- Figur 7:: eine Übersicht von Längsschnittansichten eines modularen Probeimplantatsystems umfassend drei erste und zwei zweite Probeimplantatteile;
- Figuren 8a bis 8c:: Längsschnittansichten analog den Figuren 6a bis 6c weiterer Probeimplantate eines alternativen Probeimplantatsystems;
- Figuren 9a bis 9c:: Längsschnittansichten analog den Figuren 6a bis 6c von Probeimplantaten eines weiteren Probeimplantatsystems;
- Figur 10:: eine Längsschnittansicht einer weiteren Ausführungsform eines Probeimplantats;
- Figur 11:: eine Längsschnittansicht einer weiteren Ausführungsform eines Probeimplantats;
- Figur 12:: eine Längsschnittansicht einer weiteren Ausführungsform eines Probeimplantats;
- Figur 13:: eine Längsschnittansicht einer weiteren Ausführungsform eines Probeimplantats; und
- Figur 14:: eine Längsschnittansicht einer weiteren Ausführungsform eines Probeimplantats.

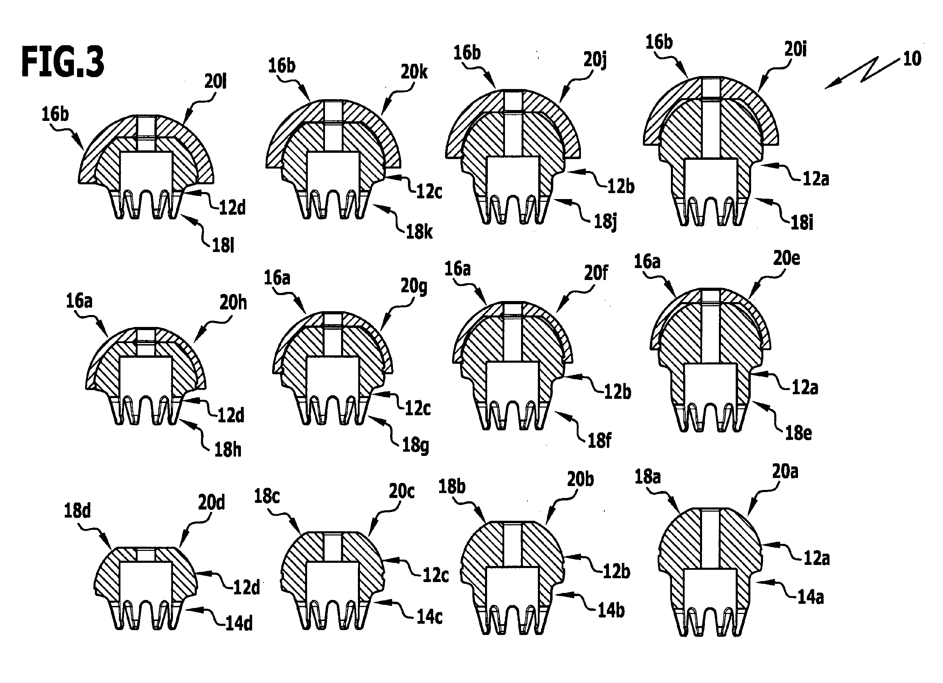

In Figur 3 ist ein insgesamt mit dem Bezugszeichen 10 versehenes modulares Probeimplantatsystem dargestellt, umfassend vier in der untersten Reihe nebeneinander dargestellte Probeimplantatteile 12a, 12b, 12c und 12d, welche Grundkörper 14a bis 14d des Probeimplantatsystems 10 bilden. Ferner umfasst das Probeimplantatsystem 10 zwei zweite Probeimplantatteile 16a und 16b, welche, wie in der mittleren und oberen Reihe in Figur 3 dargestellt, jeweils mit einem der Grundkörper 14a bis 14b lösbar verbindbar sind zur Ausbildung von Probeimplantaten 18e bis 18l. Die Grundkörper 14a bis 14d selbst bilden einstückig ausgebildete Probeimplantate 18a bis 18d, auch ohne Verbindung mit einem zweiten Probeimplantatteil 16a oder 16b.

Das Probeimplantatsystem 10 umfasst somit vier erste Probeimplantatteile 12a bis 12d und zwei zweite Probeimplantatteile 16a und 16b. Insgesamt lassen sich so, wie in Figur 3 dargestellt, durch Permutation der Komponenten zwölf Probeimplantate 18a bis 18l ausbilden.

Bei den in Figur 3 dargestellten Probeimplantaten 18a bis 18l handelt es sich um Teile eines ersten Probegelenkteils eines künstlichen Probegelenks, nämlich von Probegelenkköpfen 20a bis 20l eines künstlichen Probehüftgelenks.

Jeder erste Probeimplantatteil 12 umfasst einen im Wesentlichen kugeligen Gelenkkopfteil 22 und einen im Wesentlichen hülsenförmigen Verbindungsteil 24 mit einer in distaler Richtung weisenden, eine hohlzylindrische Halsaufnahme 26 bildenden Ausnehmung, in welche ein zapfenförmiger Halsabschnitt 28 eines Gelenkschafts 30 einführbar ist zum Verbinden des Probeimplantats 18 mit dem Gelenkschaft 30. Bei dem Gelenkschaft 30 kann es sich um einen Probegelenkschaft oder auch um den endgültig zu implantierenden Gelenkschaft handeln.

Der Gelenkkopfteil 22 ist im Wesentlichen halbkugelförmig ausgebildet, weist jedoch proximalseitig eine ebene, senkrecht zu einer Längsachse 32 des Probeimplantats 18 verlaufende Anschlagfläche 34 auf. Des Weiteren ist der Gelenkkopfteil 22 mit einer koaxial zur Längsachse 32 verlaufenden Bohrung 36 ausgestattet, welche einen Innendurchmesser aufweist, welcher etwa nur ein Drittel des Innendurchmessers der Halsaufnahme 28 beträgt.

Distalseitig wird die Halsaufnahme 26 von mehreren in distaler Richtung weisenden, fingerartig abstehenden Vorsprüngen 40 begrenzt. Eine Außenkontur des Verbindungsteils 24 verjüngt sich zum distalen Ende 38 hin leicht konisch. Des Weiteren ist ein Außendurchmesser des Verbindungsteils 24 etwas kleiner als ein Durchmesser des im Wesentlichen halbkugeligen Gelenkkopfteils 22. In einer Schnittansicht, wie insbesondere in Figur 3 gut zu erkennen, ergibt sich somit ein im Längsschnitt im Wesentlichen eine Hülsform aufweisender Grundkörper 14.

Die zweiten Implantatteile 16 sind in Form einer Gelenkkopfschale 42 ausgebildet, deren Außenkontur im Wesentlichen der einer Halbkugel entspricht. Insgesamt ist jeder zweite Probeimplantatteil 16 in Form einer Schale ausgebildet, wobei eine Schalendicke des zweiten Probeimplantatteils 16b dicker ist als die des zweiten Probeimplantatteils 16a, wie insbesondere in Figur 3 gut zu erkennen. Eine Innenkontur 44 der Gelenkkopfschale 42 ist im Wesentlichen hohlkugelig. In distaler Richtung weisend ist eine zur Anschlagfläche 34 korrespondierende ebene, einen Teil der Innenkontur 44 definierende Anschlagfläche 46 ausgebildet, die dann, wenn die ersten und zweiten Probeimplantatteile 12 und 16 miteinander verbunden sind, zumindest teilweise direkt ineinander anliegen.

Des Weiteren ist die Gelenkkopfschale 42 ebenfalls mit einer konzentrisch zur Längsachse 32 orientierten Bohrung 48 versehen, deren Innendurchmesser dem der Bohrung 36 entspricht. Die Bohrungen 36 und 48 sind derart angeordnet, dass dann, wenn die Probeimplantatteile 12 und 16 miteinander verbunden sind, ein gemeinsamer, koaxial zur Längsachse 32 verlaufender hohlzylindrischer Kanal entsteht.

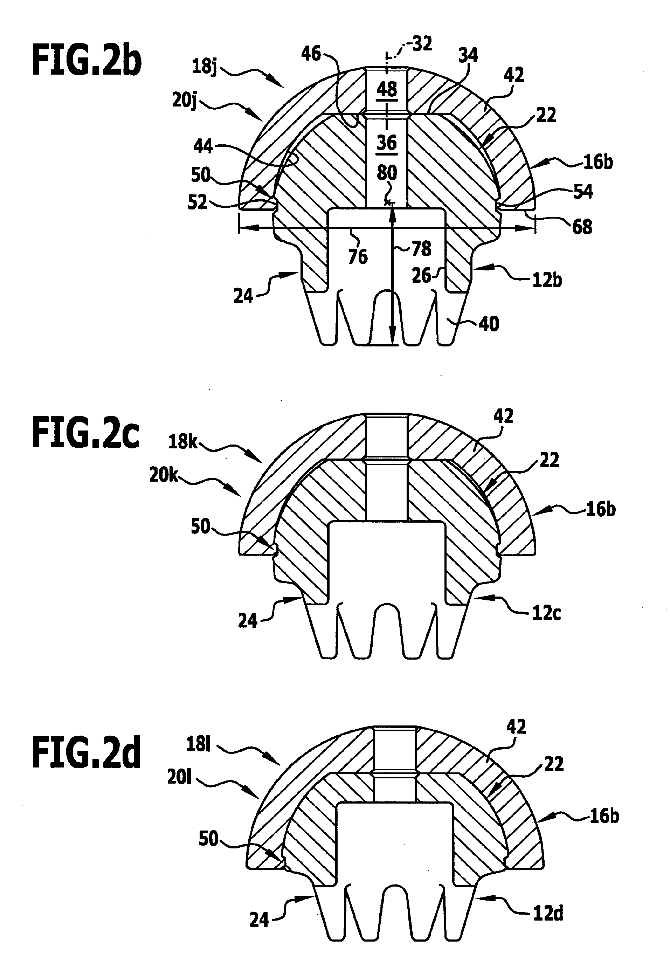

Ein Innendurchmesser der hohlkugeligen Innenkontur 44 der zweiten Probeimplantatteile 16a und 16b ist korrespondierend zu einem Außendurchmesser des kugeligen Gelenkkopfteils 22 des Grundkörpers 14d ausgebildet, so dass der Gelenkkopfteil 22 des Grundkörpers 14d im Wesentlichen formschlüssig in die von der Innenkontur 44 begrenzte Ausnehmung des zweiten Probeimplantatteils 16a beziehungsweise 16b eingreift. Dies ist insbesondere gut in Figur 2d sowie bei den in den drei Reihen links dargestellten Probeimplantaten 18d, 18h und 18l in Figur 3 zu erkennen.

Da nur zwei zweite Probeimplantatteile 16a und 16b vorgesehen sind, deren Innenkontur 44 jedoch identisch ist, liegen die Innenkontur 44 definierende Innenflächen der zweiten Probeimplantatteile 16a und 16b an den im Wesentlichen halbkugeligen Außenflächen der Gelenkkopfteile 22 der Grundkörper 14a bis 14c nicht flächig an. Um eine definierte Positionierung zu erreichen, sind hier die Anschlagflächen 34 und 46 vorgesehen, mit welchen sich eine exakte Positionierung der Gelenkkopfteile 22 bezogen auf das distale Ende 38 der Grundkörper 14 vorgeben lässt.

Zum Verbinden der ersten und zweiten Probegelenkteile 12 und 16 in einer Probeimplantationsstellung dient eine Verbindungseinrichtung 50. Die Verbindungseinrichtung 50 umfasst ein erstes Verbindungselement 52, welches am ersten Probeimplantatteil 12 angeordnet ist, sowie ein zweites Verbindungselement 54, welches am zweiten Probeimplantatteil 16 angeordnet ist. Bei dem Probeimplantatsystem 10 sind die Verbindungselemente 52 und 54 einstückig mit den Probeimplantatteilen 12 und 16 ausgebildet. Insgesamt sind die ersten und zweiten Probeimplantatteile 12 und 16 jeweils für sich einstückig ausgebildet.

Das erste Verbindungselement 52 ist in Form einer Ringnut 58 ausgebildet, welche im Wesentlichen in einer Äquatorialebene 56 des Gelenkkopfteils 22 angeordnet und von der Längsachse 32 radial nach außen weisend geöffnet ist. Die Ringnut 58 ist zudem konzentrisch zur Längsachse 32 ausgebildet. Ein Nutboden 60 der Ringnut 58 verläuft konzentrisch um die Längsachse 32, Nutseitenflächen 62 und 64, welche die Ringnut 58 seitlich begrenzen, verlaufen jeweils unter einem leichten Winkel bezogen auf die Äquatorialebene 56, so dass im Querschnitt eine im Wesentlichen wannen- oder trapezförmige Ringnut 58 ausgebildet wird.

Das zweite Verbindungselement 44 wird gebildet durch einen Ringvorsprung 66, welcher mit der Ringnut 58 in Eingriff bringbar ist. Er steht von der Innenkontur 44 in radialer Richtung auf die Längsachse 32 hin weisend im Bereich eines distalen Endes 68 des zweiten Probeimplantatteils 16 in etwa in der Äquatorialebene 56 der Gelenkkopfschale 42 vor.

Damit der Ringvorsprung 66 in die Ringnut 58 überhaupt eingreifen kann, sind ausgehend vom distalen Ende 68 gleichmäßig über einen Umfang verteilt in proximaler Richtung weisende Einschnitte 70 ausgebildet, welche zwischen sich mehrere Schalenlappen 72 definieren, welche radial nach außen etwas ausschwenken können. So ist es möglich, den zweiten Probeimplantatteil 16 auf den Gelenkkopfteil 22 des ersten Probeimplantatteils 12 aufzusetzen und in distaler Richtung zu schieben, wobei durch Aufgleiten des zweiten Verbindungselements 54 am Gelenkkopfteil 22 die Schalenlappen 72 etwas in radialer Richtung nach außen ausschwenken, jedoch dann, wenn das zweite Verbindungselement 54 die Äquatorialebene 56 erreicht, wieder in radialer Richtung auf die Längsachse 32 hin zurückfedern.

Eine im Wesentlichen in proximaler Richtung geneigte Vorsprungsseitenfläche 74 ist korrespondierend zur Nutseitenflächen 64 geneigt und liegt direkt an dieser an, wenn der Ringvorsprung 66 in die Ringnut 58 eintaucht. Es sei angemerkt, dass aufgrund der Einschnitte 70 das zweite Verbindungselement 54 in mehrere Ringvorsprungabschnitte unterteilt wird. Insgesamt ist durch die Ausbildung der ersten und zweiten Probeimplantatteile 12 und 16, wie beschrieben, ein einfaches Aufschnappen und Verrasten der Probeimplantatteile 12 und 16 in der Probeimplantationsstellung miteinander möglich. Des Weiteren können die ersten und zweiten Probeimplantatteile 12 und 16 bei Bedarf auch wieder getrennt werden.

Die Probeimplantate 18 definieren zwei Probeimplantatteilparameter 76 und 78. Der Probeimplantatteilparameter 76 definiert einen Durchmesser des Gelenkkopfteils 22. Alle Grundkörper 14 weisen denselben Außendurchmesser auf. Der zweite Probeimplantatteilparameter 78 definiert eine Verbindungsteil oder Halslänge 78, welche definiert wird durch einen Abstand des distalen Endes 38 des ersten Probeimplantatteils 12 von einem Mittelpunkt 80 des Gelenkkopfteils 72 parallel zur Längsachse 32. Beim Probeimplantatsystem 10 unterscheiden sich die Grundkörper 14a bis 14d jeweils im zweiten Probeimplantatteilparameter 78. Dieser ist am größten beim Grundkörper 14a und nimmt stufenweise bis zum Grundkörper 14d ab. Der erste Probeimplantatteilparameter 76 kann beispielsweise einen Durchmesser von 28mm, 32mm und 36mm definieren. Diese Außendurchmesser werden definiert durch die Gelenkkopfteile 22 der Grundkörper 14a bis 14d beziehungsweise durch die zweiten Probeimplantatteile 16a und 16b.

Insgesamt ist es mit den zur Verfügung stehenden vier ersten Probeimplantatteilen 12a bis 12d sowie den zwei zweiten Probeimplantatteilen 16a und 16b möglich, insgesamt zwölf verschiedene Probeimplantate 18a bis 18l zu kombinieren. Es sind also nur halb so viele Komponenten erforderlich als bei insgesamt einstückig ausgebildeten Probeimplantaten, mit welchen die möglichen Kombinationen der sieben verschiedenen Probeimplantatteilparameter 76 und 78 kombinierbar sind.

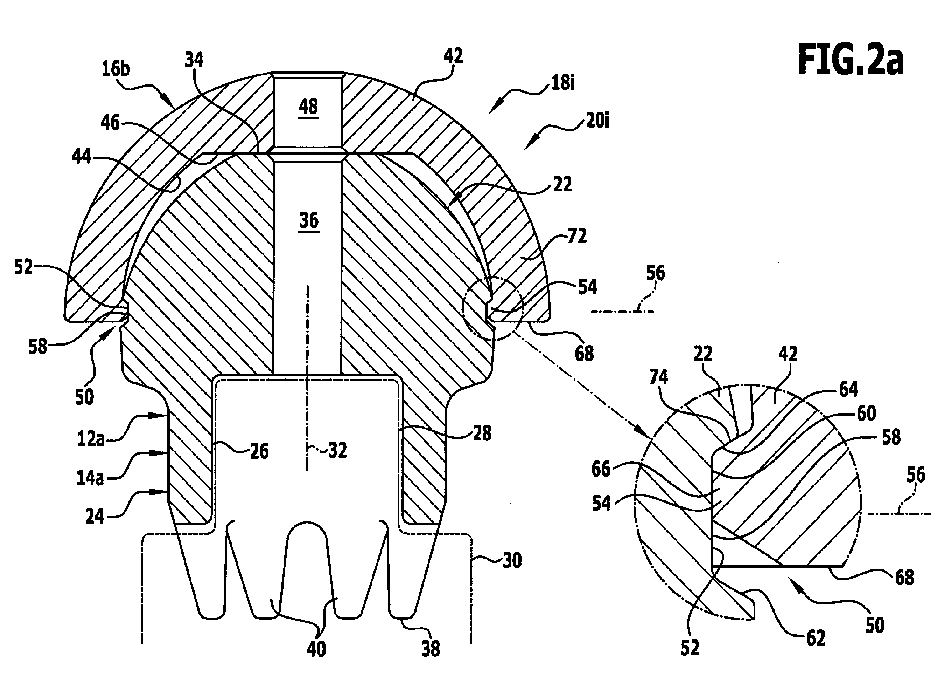

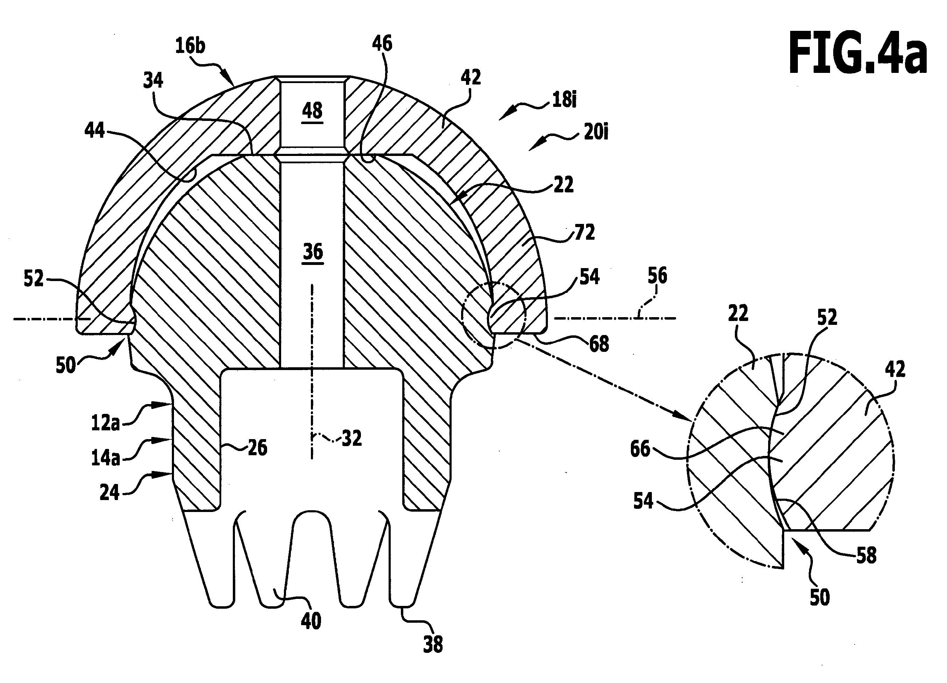

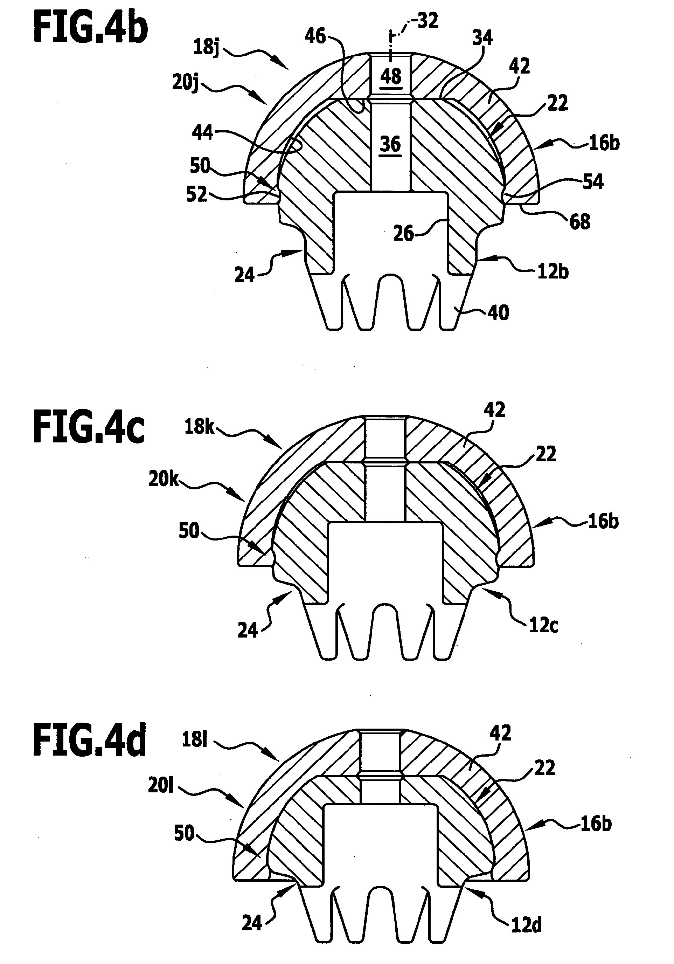

Die in den Figuren 4a bis 4d dargestellten Probeimplantaten 18i bis 18l stimmen in ihrem Grundaufbau mit den in den Figuren 2a bis 2d dargestellten Probeimplantaten 18i bis 18l überein. Sie unterscheiden sich lediglich im Aufbau der Verbindungseinrichtung 50. Das zweite Verbindungselement 54 ist bei der in den Figuren 4a bis 4d dargestellten Variante im Querschnitt nicht trapezförmig, sondern als eine halblinsenförmiger, in Richtung auf die Längsachse 32 hin weisender, konvex gekrümmter Ringvorsprung ausgebildet. Das erste Verbindungselement 52 ist entsprechend in Form einer Ringnut 58 ausgebildet, welche von der Längsachse 32 weg in radialer Richtung weisend hohlkonkav gekrümmt ist. Auch diese Ausgestaltung der Verbindungseinrichtung 50 ermöglicht die Ausbildung einer Rast- oder Schnappverbindung zur Verbindung der ersten und zweiten Probeimplantatteile 12 und 16 miteinander.

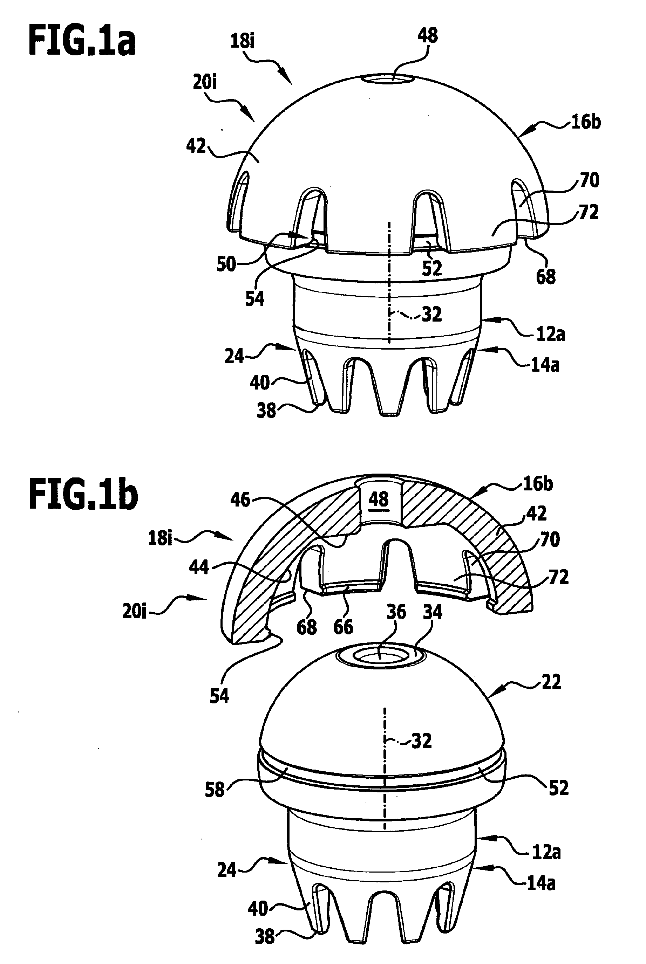

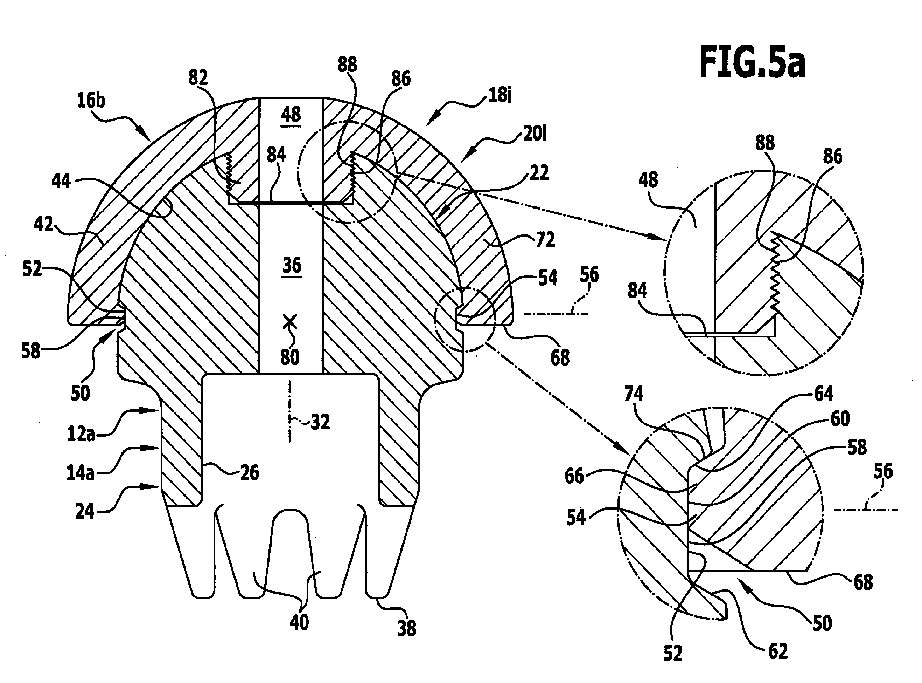

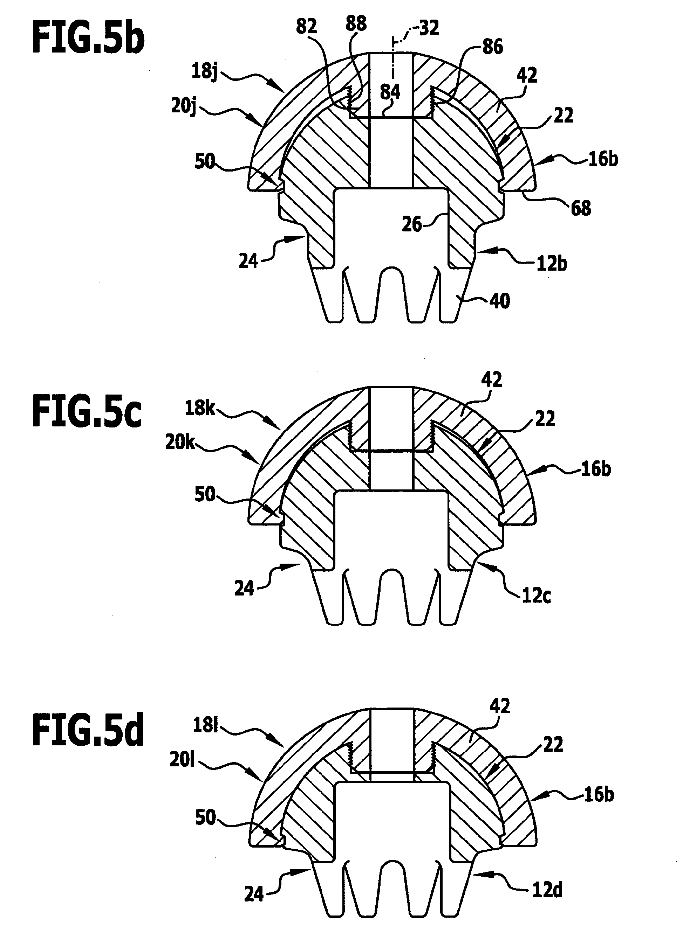

Bei den in den Figuren 1 bis 3 dargestellten Probeimplantaten 18 dienen die Anschlagflächen 34 und 46 dazu, den Mittelpunkt 80 zwischen den Grundkörper 14 und den Gelenkkopfschalen 42 zu korrigieren. Dies ist erforderlich, da ein Linearitätssprung zwischen den einzelnen Halslängen von Durchmesser zu Durchmesser der Gelenkkopfteil 22 variiert. Alternativ können unterschiedliche oder auch andere Formen von Abstandshaltern vorgesehen sein, beispielsweise Gewinde, die bereits beschriebenen Anschlagflächen 34 und 46 oder auch nur in eine Ausnehmung eintauchende Zapfen. Dies ist beispielhaft in den Figuren 5a bis 5d dargestellt. Bei den in den Figuren 5a bis 5d dargestellten zweiten Probeimplantatteilen 16b ist keine Anschlagfläche 46 vorgesehen, sondern stattdessen ein parallel zur Längsachse 32 in distaler Richtung abstehender Zapfen 82, welcher in eine korrespondierend ausgebildete und in proximaler Richtung weisende, konzentrisch zur Längsachse 32 ausgebildete Ausnehmung des Gelenkkopfteils 22 ausgebildet ist. Optional kann zur Sicherung einer Verbindung beziehungsweise zum Einstellen einer Position des Mittelpunkts 80 optional eine Gewindeverbindung vorgesehen sein, welche ein Außengewinde 86 am Zapfen 82 und ein Innengewinde 88 an der Aufnahme 84 umfasst, die korrespondierend zueinander ausgebildet sind und ein Verschrauben der ersten und zweiten Probeimplantatteile 12 und 16 miteinander ermöglichen. Die Verbindungseinrichtung 50 ist bei dem in den Figuren 5a bis 5d dargestellten Ausführungsbeispiel identisch mit der in den Figuren 2a bis 2d dargestellten Verbindungseinrichtung 50 ausgebildet.

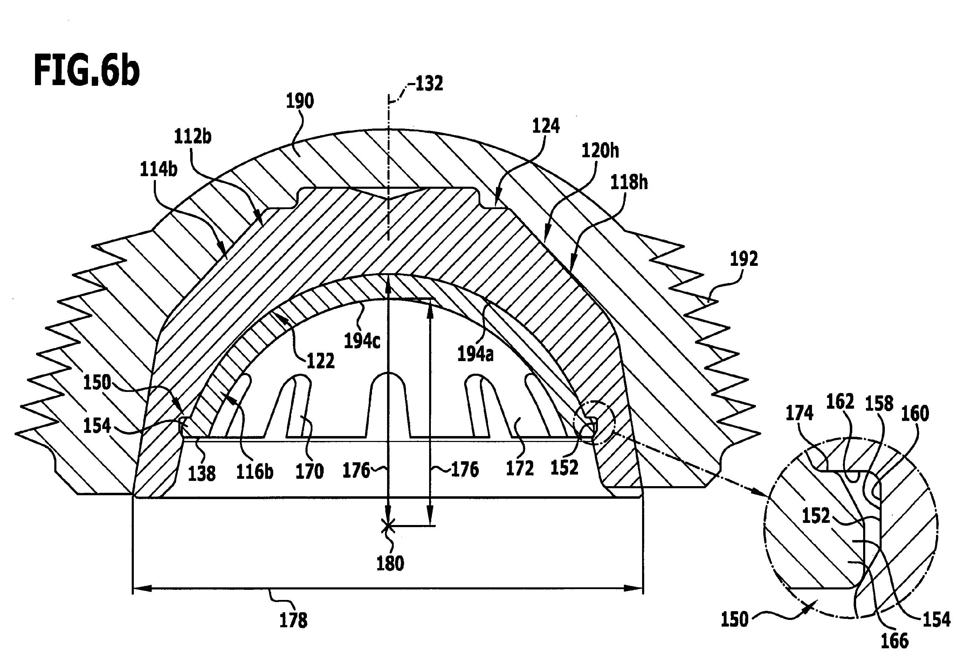

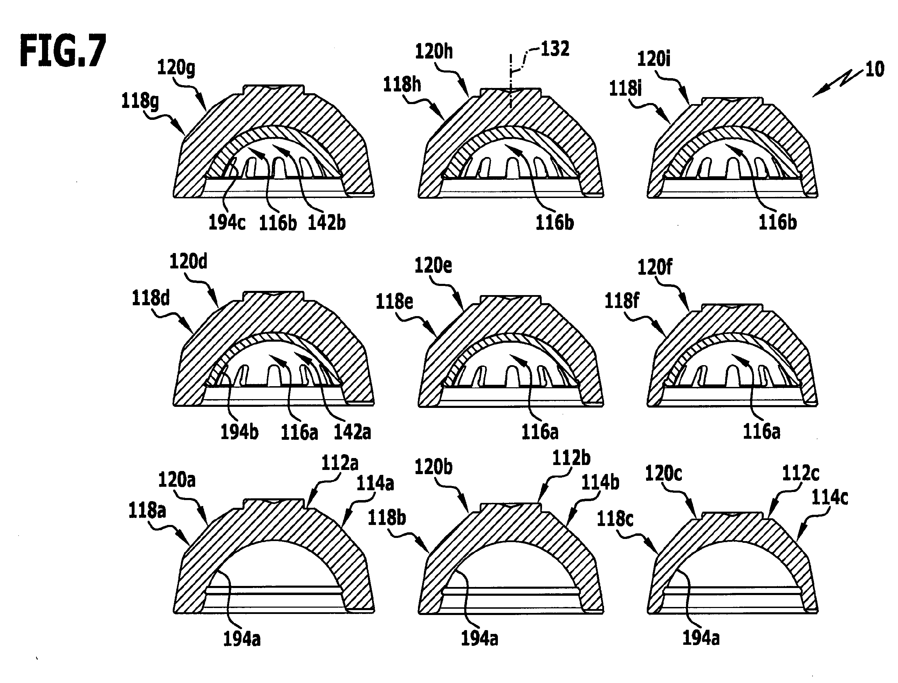

Das Probeimplantatsystem 10 kann ferner optional oder alternativ auch Probegelenkpfanneneinsätze 120 umfassen, beispielsweise die in Figur 7 dargestellten Pfanneneinsätze 120a bis 120i. Sie sind, wie beispielhaft in Figur 6b für den Pfanneneinsatz 120e gezeigt, mit einer Gelenkpfanne 190, insbesondere einer Probegelenkpfanne oder einer endgültig in den Körper eines Patienten zu implantierenden Gelenkpfanne, verbunden oder verbindbar, und zwar in bekannter Weise. Bei der Gelenkpfanne 190 kann es sich insbesondere um eine Schraubpfanne mit einem Schraubgewinde 192 handeln, welche in einen nicht dargestellten Beckenknochen eines Patienten eingeschraubt werden kann. Die Pfanneneinsätze 120 umfassen einen Pfanneneinsatzteil 122 sowie einen Pfannenverbindungsteil 124. Der Pfannenverbindungsteil 124 dient insbesondere dazu, den jeweiligen Pfanneneinsatz 120 mit der Gelenkpfanne 190 zu verbinden.

Die Modularität der Pfanneneinsätze 120 entspricht dem im Zusammenhang mit den oben beschriebenen Probeimplantaten 18 erläuterten Prinzip. Die ersten Probeimplantatteile 112a, 112b und 112c, wie in der untersten Reihe in Figur 7 dargestellt, definieren jeweils zwei Probeimplantatteilparameter 176 und 178. Der Probeimplantatteilparameter 176 definiert einen Innendurchmesser einer Gelenkfläche 194a bezogen auf einen Mittelpunkt 180, der dann, wenn der Pfanneneinsatz 120 mit einem der oben beschriebenen Probegelenkköpfe 20 verbunden ist, mit dem Mittelpunkt 80 zusammenfällt. Der weitere Probeimplantatteilparameter 178 definiert eine Außenform beziehungsweise einen Außendurchmesser des ersten Probeimplantatteils 112. Der Probeimplantatteilparameter 178 ist beim ersten Probeimplantatteil 112a größer als beim Probeimplantatteil 112c. Der Pfanneneinsatz 120c ist somit für kleine Gelenkpfannen 190 geeignet, der Pfanneneinsatz 120b für mittlere und der Pfanneneinsatz 120a für große Gelenkpfannen 190.

Zweite Probeimplantatteile 116a und 116b bilden Einsatzverkleinerungsteile 142a und 142b. Mit ihnen ist es möglich, durch Verbinden mit den Pfanneneinsatzteilen 122 der ersten Probeimplantatteilen 112 den Probeimplantatteilparameter 176 zu variieren, das heißt den Innendurchmesser der von der Gelenkfläche 194a definierten Kavität. Die Innendurchmesser sind korrespondierend zu den Probeimplantatteilparametern 76 ausgebildet, das heißt beispielsweise beträgt ein Innendurchmesser bei den Grundkörper 114a bis 114c 36mm, beim Einsatzverkleinerungsteil 142a 32mm und beim Einsatzverkleinerungsteil 142b 28mm.

Insgesamt können somit mit drei Grundkörpern 114a bis 114c und zwei Einsatzverkleinerungsteilen 142a und 142b neun verschiedene Pfanneneinsätze 120 gebildet werden, wie in Figur 7 dargestellt.

Zum Verbinden der zweiten Probeimplantatteile 116 mit den ersten Probeimplantatteilen 112 dient wiederum eine Verbindungseinrichtung 150 mit ersten und zweiten Verbindungselementen 152 und 154, die an den ersten beziehungsweise zweiten Probeimplantatteilen 112 und 116 angeordnet beziehungsweise ausgebildet sind. Das erste Verbindungselement 152 wird wiederum definiert durch eine in Richtung auf die Längsachse 132 der Probeimplantate 118a bis 118i hin weisende Ringnut 158, welche eine quer zur Längsachse 132 verlaufende Nutseitenfläche 162 aufweist. Die Nutseitenfläche 162 bildet eine Rastfläche für eine in proximaler Richtung weisende Vorsprungsseitenfläche 174 eines das zweite Verbindungselement 154 bildenden Ringvorsprungs 168 am zweiten Probeimplantatteil 116. Um die Rast- beziehungsweise Schnappverbindung zwischen den ersten und zweiten Probeimplantatteilen 116 und 118 zu realisieren, sind wiederum ausgehend von einem distalen Ende des zweiten Probeimplantatteils 116 in proximaler Richtung verlaufende Einschnitte 170 ausgebildet, die zwischen sich Schalenlappen 172 an den Einsatzverkleinerungsteil 142 definieren, so dass der Ringvorsprung 166 in einzelne Ringvorsprungabschnitte aufgeteilt wird, die jedoch in radialer Richtung etwas beweglich sind. Dies ermöglicht, beim Einführen der Einsatzverkleinerungsteile 142 in die durch die Gelenkflächen 194 begrenzten Aufnahmen, ein Aufgleiten und Einrasten der zweiten Verbindungselemente 154 in die ersten Verbindungselemente 152.

Grundgedanke ist demnach bei allen Probeimplantaten 118, eine modulare Probepfanne auszubilden, wobei der größte Durchmesser der Artikulationspartner durch die Einsatzverkleinerungsteile 142 verkleinerbar ist. Die Verbindung der ersten und zweiten Probeimplantatteile 112 und 116 kann, wie beschrieben, durch Einschnappen oder Verrasten erfolgen, jedoch auch durch Einschrauben oder Verschrauben und/oder durch ein anderes beliebiges lösbares Verbindungsverfahren. Entsprechende Verschiebungen der Mittelpunkte 180 werden zwischen den unterschiedlichen Größen der Einsatzverkleinerungsteile 142 durch diese selbst realisiert.

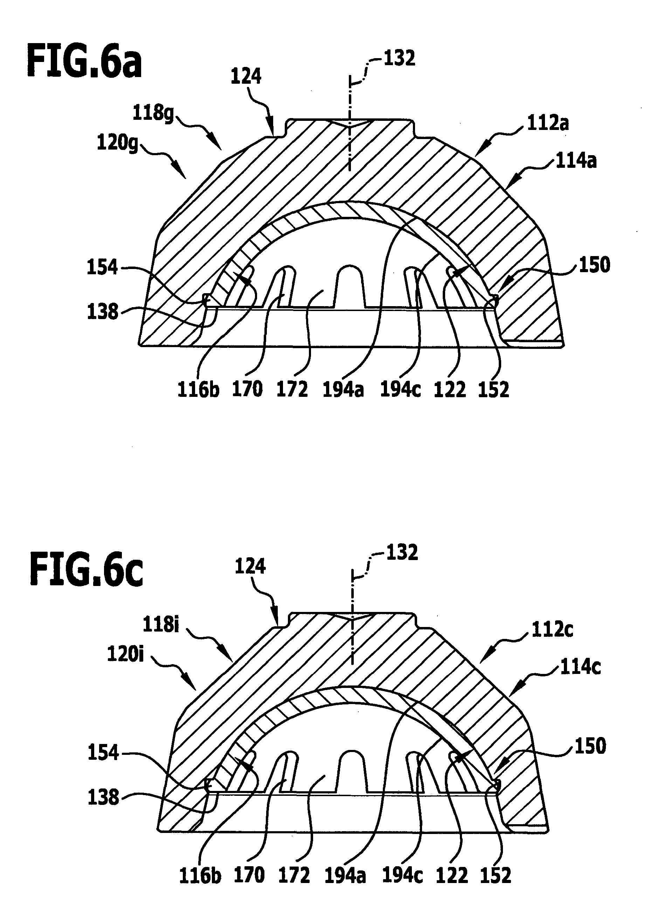

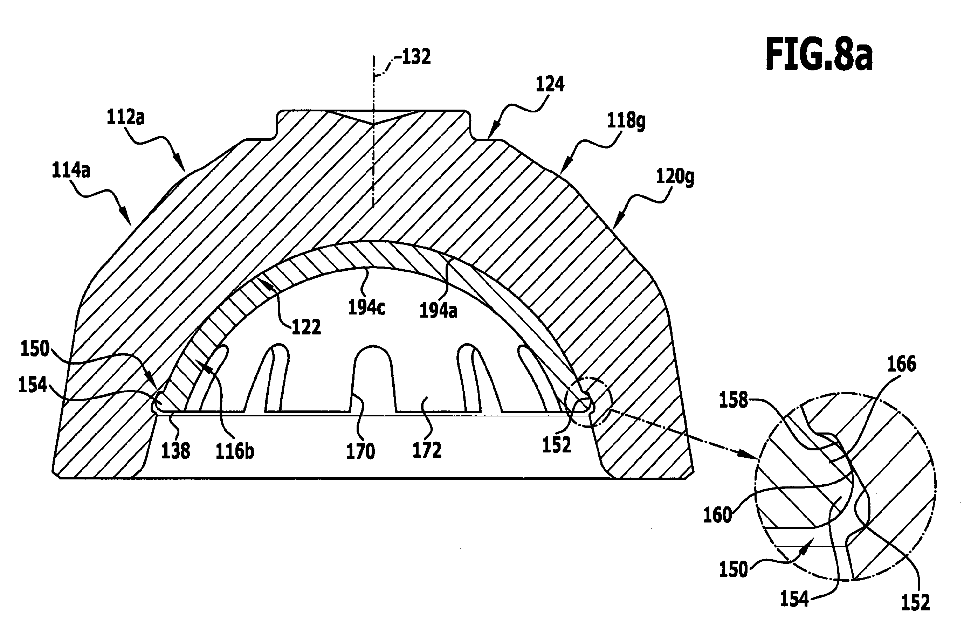

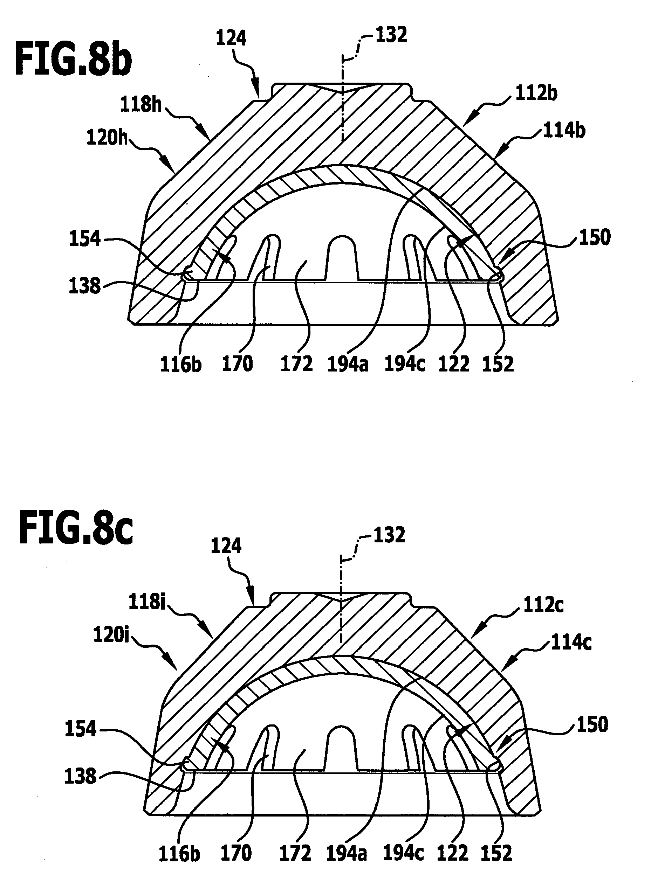

Bei den in den Figuren 8a bis 8c dargestellten Pfanneneinsätzen 120d bis 120f ist die Verbindungseinrichtung 150 etwas von der bei den Probeimplantaten 118 in den Figuren 6a bis 6c vorgesehene abweichend ausgebildet. Das zweite Verbindungselement 154 ist in Form eines ringwulstförmigen Vorsprungs 166 ausgebildet, das erste Verbindungselement 152 wiederum in Form einer Ringnut 158, wobei jedoch ein Nutboden 160, anders als der konzentrisch zur Längsachse 132 orientierte bei den in den Figuren 6a bis 6c dargestellten Ausführungsbeispielen, relativ zur Längsachse 132 geneigt ist und etwas in distaler Richtung weist. Ansonsten unterscheiden sich die in den Figuren 6 und 8 dargestellten Probeimplantate 118 nicht.

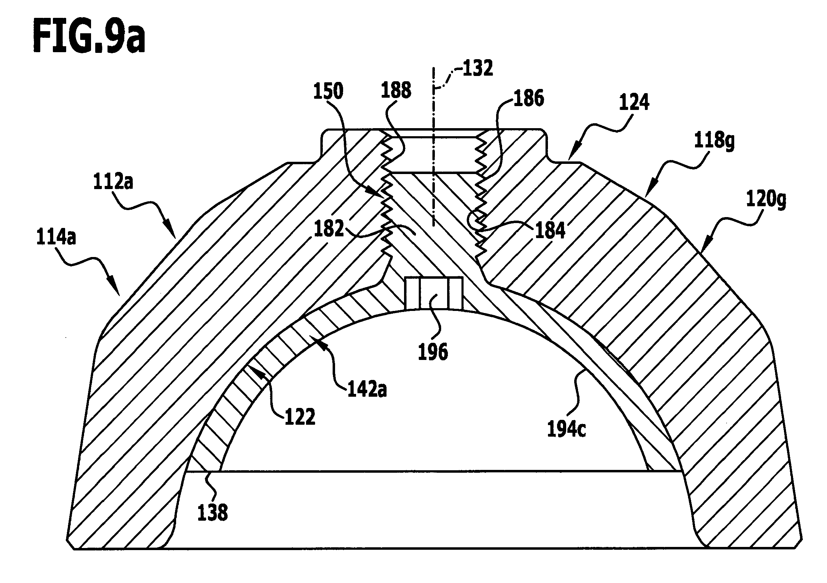

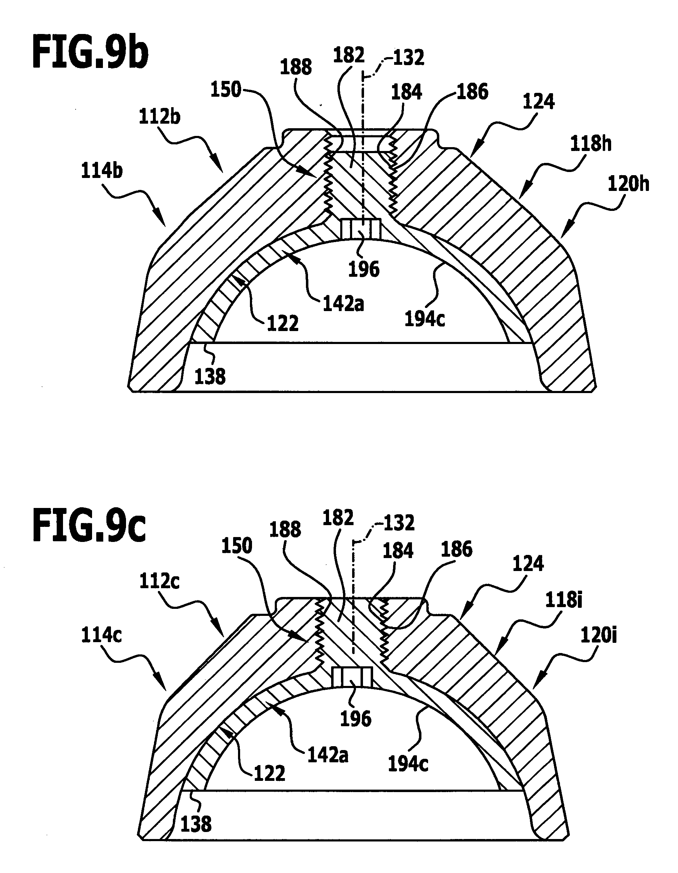

In den Figuren 9a bis 9c sind drei weitere Pfanneneinsätze 120d bis 120f dargestellt, die sich wiederum im Wesentlichen nur durch die Ausbildung der Verbindungseinrichtung 150 unterscheiden. Hier ist jedoch keine Schnappverbindung oder Rastverbindung vorgesehen, sondern eine Schraubverbindung. Am Einsatzverkleinerungsteil 142 ist jeweils ein in proximaler Richtung konzentrisch zur Längsachse 132 abstehender Zapfen 182 ausgebildet, welcher in eine korrespondierende, hohlzylindrische Aufnahme 184 am Grundkörper 114 eingreift. Der Zapfen 182 und die Aufnahme 184 können durch entsprechende Bemaßung bereits ausreichend für eine Klemmung ausgebildet sein. Um eine dauerhafte und sichere Verbindung zu gewährleisten, ist der Zapfen 182 mit einem Außengewinde 186 und die Aufnahme 184 mit einem zum Außengewinde 186 korrespondierenden Innengewinde 188 versehen, so dass die ersten und zweiten Probeimplantatteile 112 und 116 auf einfache Weise miteinander verschraubt werden können. Um das Verbinden zu erleichtern, ist eine in distaler Richtung weisende Werkzeugaufnahme 196 in die Gelenkfläche 194c eingearbeitet, vorzugsweise in Form eines Innensechskant oder eines Innenvielrund, in welche ein korrespondierendes Ende eines nicht dargestellten Einschraubwerkzeugs eingreifen kann.

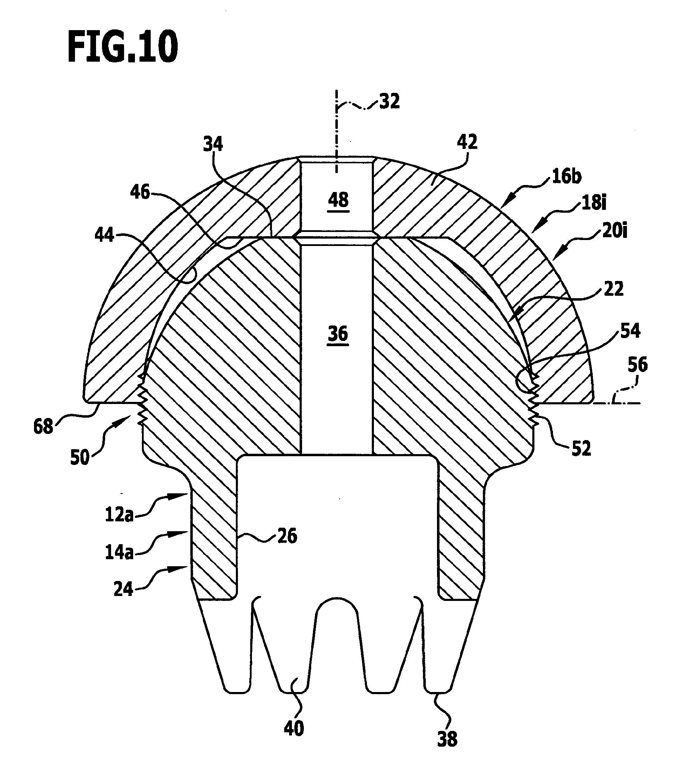

Alternativ können die Probeimplantate 18 wie bei dem in Figur 10 beispielhaft dargestellten Probeimplantat 18i auch miteinander verschraubt sein. Hierfür ist eine Verbindungseinrichtung 50 vorgesehen, welche erste und zweite Verbindungselemente 52 und 54 umfasst, und zwar in Form von Innengewinde- und Außengewindeabschnitten, wobei insbesondere ausgehend vom distalseitigen Ende 68 des zweiten Probeimplantatteils 16 ein Innengewindeabschnitt ausgebildet ist. Ein korrespondierender Außengewindeabschnitt ist in etwa im Bereich der Äquatorialebene 56 an einem Probeimplantatteil ausgebildet, so dass die beiden Probeimplantatteile 12 und 16 miteinander verschraubt werden können. Ansonsten sind die Probeimplantate 18 analog den in Figur 7 dargestellten ausgebildet.

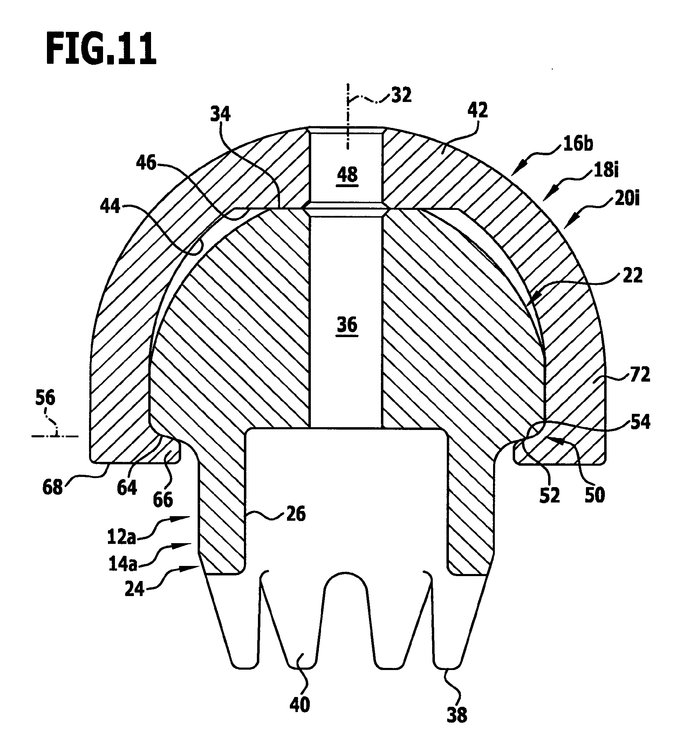

Eine alternative Schnappverbindung zwischen den ersten und zweiten Probeimplantatteilen 12 und 16 ist in Figur 11 beispielhaft am Probeimplantat 18i dargestellt. Die Verbindungseinrichtung 50 umfasst erste und zweite Verbindungselemente 52 und 54, und zwar eine Rastkante, welche eine im Wesentlichen in distaler Richtung weisende Anlagefläche definiert sowie von den Schalenlappen 72 abstehende und in radialer Richtung auf die Längsachse 32 hin weisenden Rastnase 66, die in einer Probeimplantationsstellung, in welcher die Probeimplantatteile 12 und 16 miteinander verbunden sind, wie in Figur 11 dargestellt, die Rastkante 64 hintergreifen.

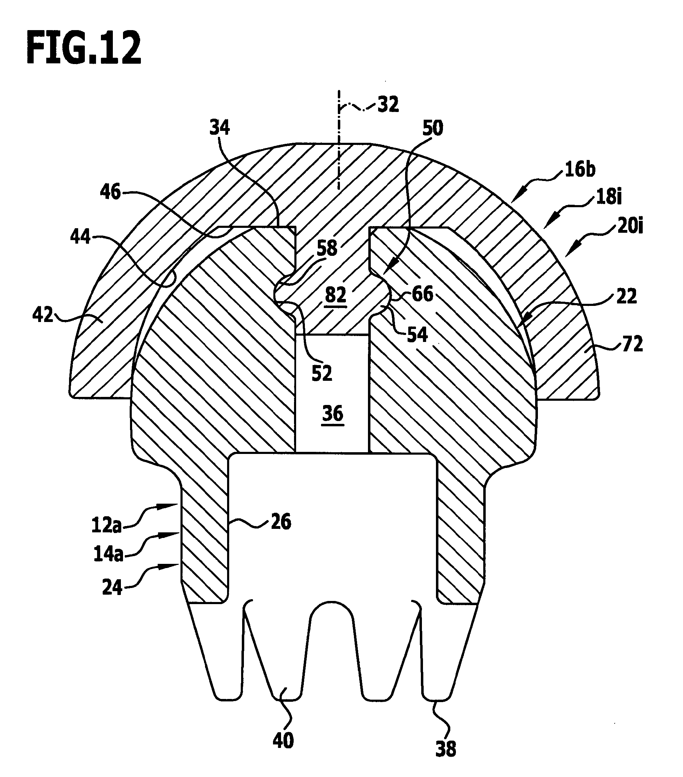

Eine weitere Art der Verbindung zwischen den ersten Probeimplantationsteilen 12 und 16 ist in Figur 12 beispielhaft am Probeimplantat 18i dargestellt. Ein vom zweiten Probeimplantatteil 16 in distaler Richtung weisend abstehender Zapfen 82 ist korrespondierend zur Bohrung 36 am ersten Probeimplantatteil 12a ausgebildet. Zur Verbindung der beiden Teile in der Probeimplantationsstellung dient die Verbindungseinrichtung 50, welche einen radial von der Längsachse 32 weg weisenden, ringwulstartigen Vorsprung 66 am Zapfen 82 aufweist, welcher in der Probeimplantationsstellung in eine korrespondierende Ringnut 56, die ein zweites Verbindungselemente 54 definiert, an der Bohrung eingreift. Weitere Verbindungselemente sind zur Verbindung der Probeimplantationsteile in diesem Fall nicht erforderlich.

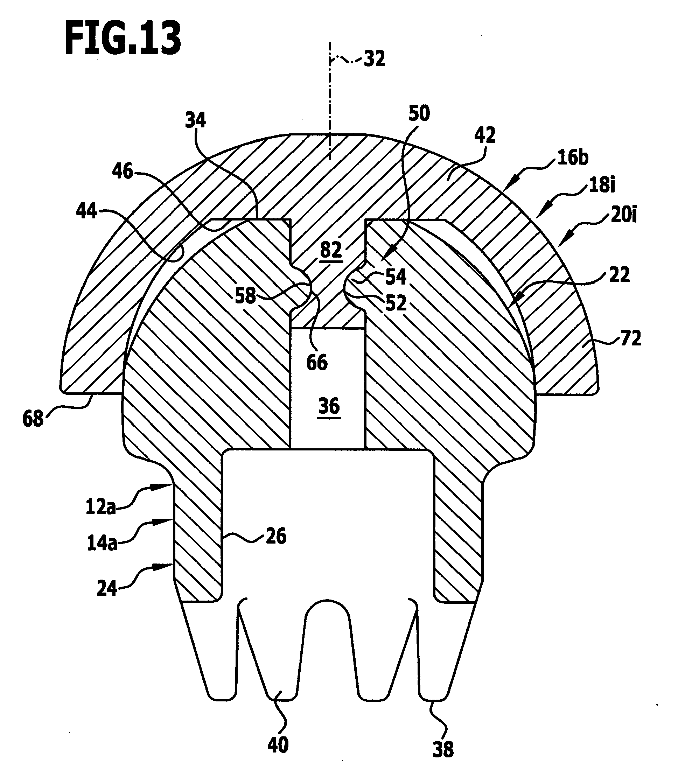

Eine Variante der im Zusammenhang mit Figur 12 beschriebenen Verbindungseinrichtung 50 ist bei dem in Figur 13 beispielhaft dargestellten Probeimplantat 18i verwirklicht. In diesem Fall bildet das erste Verbindungselement 52 eine Ringnut am Zapfen 82, in welche ein auf die Längsachse 32 hin weisender ringwulstförmiger, an der Bohrung 36 angeordneter Vorsprung 66 des ersten Probeimplantats 12 in der Probeimplantationsstellung eingreift.

An dieser Stelle sei angemerkt, dass, wo dies mechanisch möglich ist, die miteinander in Verbindung stehenden und insbesondere in der Probeimplantationsstellung in Eingriff stehenden Verbindungselemente der Verbindungseinrichtungen 50 beziehungsweise 150 wahlweise am einen oder am anderen Probeimplantatteil angeordnet sein können. Insofern sind die im Zusammenhang mit den Figuren beschriebenen Ausführungsbeispiele rein beispielhaft.

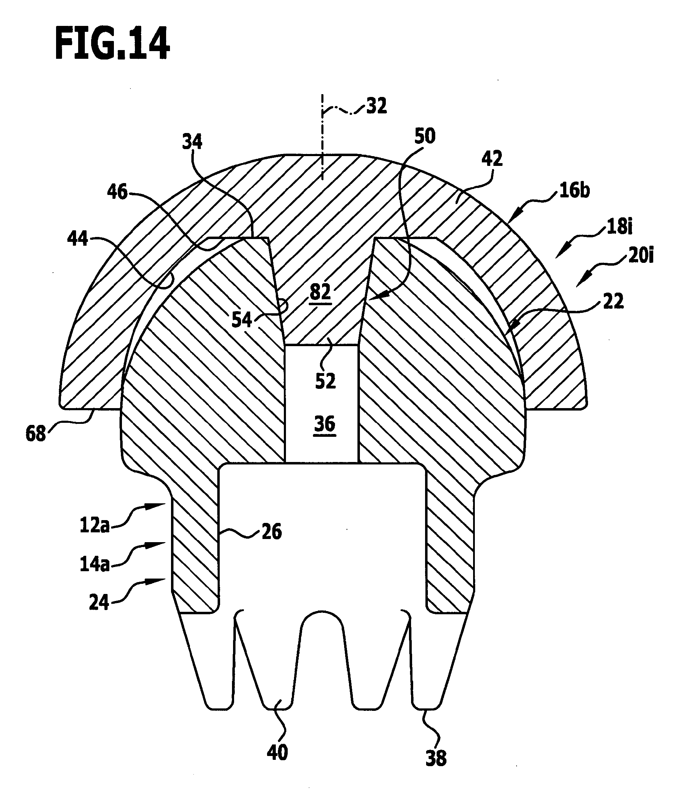

Abschließend wird eine weitere Variante einer Verbindungseinrichtung beispielhaft im Zusammenhang mit den in Figur 14 dargestellten Probeimplantat 18i beschrieben und dargestellt. Der bei den Varianten, wie sie in den Figuren 12 und 13 dargestellt sind, vorgesehene Zapfen 82 verjüngt sich in distaler Richtung schwach konisch. Umgekehrt erweitert sich die Bohrung 36 zu ihrem proximalseitigen Ende hin korrespondierend schwach konisch. Dies ermöglicht es, den Zapfen 82 in die sich folglich in distaler Richtung ebenfalls konisch verjüngende Bohrung 36 einzusetzen, bis eine Verbindung über einen Reibschluss erreicht wird. Man macht sich also eine Konusklemmung zu Nutze, um die ersten und zweiten Probeimplantatteile 12 und 16 miteinander in der Probeimplantationsstellung zu verbinden.

Die im Zusammenhang mit den Figuren 10 bis 14 beschriebenen Verbindungseinrichtungen sind analog im Prinzip auch auf die Pfanneneinsätze 120 übertragbar. Mit anderen Worten könnten die Verbindungseinrichtungen 150 analog wie die im Zusammenhang mit den Figuren 10 bis 14 beschriebenen Verbindungseinrichtungen 50 ausgebildet sein.

Die Probeimplantate 18 können ganz oder teilweise aus Metall oder Kunststoff hergestellt sein. Vorzugsweise sind die Probeimplantatteile 12 und 16 jeweils einstückig ausgebildet.

## Patentansprüche

1. Modulares Probeimplantatsystem (10) umfassend mindestens ein Probeimplantat (18; 118), welches mindestens einen ersten und mindestens einen zweiten, mit dem mindestens einen ersten Probeimplantatteil (12; 112) verbindbaren Probeimplantatteil (16; 116) umfasst, wobei mindestens eine Verbindungseinrichtung (50; 150) zum Verbinden des mindestens einen ersten und des mindestens einen zweiten Probeimplantatteils (12, 16; 112, 116) in einer Probeimplantationsstellung vorgesehen ist, wobei die mindestens eine Verbindungseinrichtung (50; 150) mindestens ein erstes und mindestens ein zweites Verbindungselement (52, 54; 152, 154) umfasst, wobei das eine Verbindungselement (52; 152) am mindestens einen ersten Probeimplantatteil (12; 112) und wobei das andere Verbindungselement (54; 154) am mindestens einen zweiten Probeimplantatteil (16; 116) angeordnet ist und wobei das mindestens eine erste und das mindestens eine zweite Verbindungselement (52, 54; 152, 154) korrespondierend zueinander ausgebildet sind und in der Probeimplantationsstellung in Eingriff stehen, **dadurch gekennzeichnet, dass** die mindestens eine Verbindungseinrichtung (50; 150) in Form einer Rastverbindung ausgebildet ist.

2. Probeimplantatsystem nach Anspruch 1, **dadurch gekennzeichnet, dass** der mindestens eine erste und der mindestens eine zweite Probeimplantatteil (12, 16; 112, 116) miteinander lösbar verbindbar sind.

3. Probeimplantatsystem nach Anspruch 1 oder 2, **dadurch gekennzeichnet, dass** das mindestens eine erste und/oder das mindestens eine zweite Verbindungselement (52, 54; 152, 154) in Form zueinander korrespondierender Rastelemente (58, 66; 158, 166) ausgebildet sind.

4. Probeimplantatsystem nach Anspruch 3, **dadurch gekennzeichnet, dass** das eine Rastelement (58; 158) in Form einer Ringnut (58; 158) oder eines Ringnutabschnitts und **dass** das korrespondierende andere Rastelement (66; 166) in Form eines Ringwulstes (66; 166) oder eines Ringwulstabschnitts ausgebildet ist, welcher Ringwulst (66; 166) oder Ringwulstabschnitt in der Probeimplantationsstellung in die Ringnut (58; 158) oder den Ringnutabschnitt eintaucht oder eingreift.

5. Probeimplantatsystem nach einem der voranstehenden Ansprüche, **dadurch gekennzeichnet, dass** das mindestens eine erste und/oder das mindestens eine zweite Verbindungselement (52, 54; 152, 154) in Form von korrespondierenden Aufnahmen (36; 58; 158) und Vorsprüngen (66; 82; 166) ausgebildet sind, die in der Probeimplantationsstellung kraft- und/oder formschlüssig miteinander verbunden sind.

6. Probeimplantatsystem nach Anspruch 5, **dadurch gekennzeichnet, dass** das mindestens eine erste und das mindestens eine zweite Rastelement (52, 54) an den korrespondierenden Aufnahmen (36) und Vorsprüngen (82) angeordnet sind.

7. Probeimplantatsystem nach einem der voranstehenden Ansprüche, **dadurch gekennzeichnet, dass** die Verbindungseinrichtung (50; 150) rotationssymmetrisch bezogen auf eine Probeimplantatlängsachse (32; 132) ausgebildet ist.

8. Probeimplantatsystem nach einem der voranstehenden Ansprüche, **dadurch gekennzeichnet, dass** das mindestens eine erste Probeimplantatteil (12; 112) einen Grundkörper (14; 114) des Probeimplantatsystems (10) bildet.

9. Probeimplantatsystem nach einem der voranstehenden Ansprüche, **dadurch gekennzeichnet, dass** das mindestens eine erste Probeimplantatteil (12; 112) einstückig ausgebildet ist.

10. Probeimplantatsystem nach einem der voranstehenden Ansprüche, **dadurch gekennzeichnet, dass** das mindestens eine zweite Probeimplantatteil (16; 116) einstückig ausgebildet ist.

11. Probeimplantatsystem nach einem der voranstehenden Ansprüche, gekennzeichnet durch mindestens zwei unterschiedlich geformte erste Probeimplantatteile (12; 112).

12. Probeimplantatsystem nach einem der voranstehenden Ansprüche, gekennzeichnet durch mindestens zwei unterschiedlich geformte zweite Probeimplantatteile (16; 116).

13. Probeimplantatsystem nach einem der voranstehenden Ansprüche, **dadurch gekennzeichnet, dass** der mindestens eine erste Probeimplantatteil (12; 112) mindestens zwei Probeimplantatteilparameter (76, 78; 176, 178) definiert.

14. Probeimplantatsystem nach Anspruch 13, **dadurch gekennzeichnet, dass** sich die mindestens zwei unterschiedlich geformten ersten Probeimplantatteile (12; 112) in mindestens einem ersten Probeimplantatteilparameter (76; 176) unterscheiden.

15. Probeimplantatsystem nach einem der voranstehenden Ansprüche, **dadurch gekennzeichnet, dass** der mindestens eine zweite Probeimplantatteil (16; 116) mindestens zwei Probeimplantatteilparameter (76, 78; 176, 178) definiert.

16. Probeimplantatsystem nach Anspruch 15, **dadurch gekennzeichnet, dass** sich die mindestens zwei unterschiedlich geformten zweiten Probeimplantatteile (16; 116) in mindestens einem zweiten Probeimplantatteilparameter (76, 78; 176, 178) unterscheiden.

17. Probeimplantatsystem nach einem der voranstehenden Ansprüche, gekennzeichnet durch mindestens ein Probeimplantat (18) in Form eines Probegelenkkopfs (20), welcher einen Gelenkkopfteil (22) und einen Verbindungsteil (24) umfasst.

18. Probeimplantatsystem nach Anspruch 17, **dadurch gekennzeichnet, dass** der Verbindungsteil (24) eine Halsaufnahme (26) umfasst, in welche ein zapfenförmiger Halsabschnitt (28) eines Gelenkschafts (30) einführbar ist zum Verbinden des Probegelenkkopfs (20) mit dem Gelenkschaft (30).

19. Probeimplantatsystem nach einem der voranstehenden Ansprüche, **dadurch gekennzeichnet, dass** der mindestens eine zweite Probeimplantatteil (16) in Form einer Gelenkkopfschale (42) ausgebildet ist.

## Claims

1. Modular trial implant system (10) comprising at least one trial implant (18; 118) which comprises at least one first trial implant part (12; 112) and at least one second trial implant part (16; 116) connectable to the at least one first trial implant part (12; 112), wherein at least one connecting device (50; 150) is provided for connecting the at least one first and the at least one second trial implant part (12, 16; 112, 116) in a trial implantation position, wherein the at least one connecting device (50; 150) comprises at least one first and at least one second connecting element (52, 54; 152, 154), wherein the one connecting element (52; 152) is arranged at the at least one first trial implant part (12; 112) and wherein the other connecting element (54; 154) is arranged at the at least one second trial implant part (16; 116) and wherein the at least one first and the at least one second connecting element (52, 54; 152, 154) are configured in corresponding relationship to each other and engage with each other in the trial implantation position, **characterized in that** the at least one connecting device (50; 150) is configured in the form of a latch connection.

2. Trial implant system in accordance with claim 1, **characterized in that** the at least one first and the at least one second trial implant part (12, 16; 112, 116) are releasably connectable to each other.

3. Trial implant system in accordance with claim 1 or 2, **characterized in that** the at least one first and/or the at least one second second connecting element (52, 54; 152, 154) are/is configured in the form of latch elements (58, 66; 158, 166) which correspond to each other.

4. Trial implant system in accordance with claim 3, **characterized in that** the one latch element (58; 158) is configured in the form of an annular groove (58; 158) or a section of annular groove and **in that** the corresponding other latch element (66; 166) is configured in the form of an annular bead (66; 166) or a section of annular bead, which annular bead (66; 166) or section of annular bead extends into or engages in the annular groove (58; 158) or section of annular groove when in the trial implantation position.

5. Trial implant system in accordance with any one of the preceding claims, **characterized in that** the at least one first and/or the at least one second connecting element (52, 54; 152, 154) are/is configured in the form of corresponding receptacles (36; 58; 158) and projections (66; 82; 166) which, when in the trial implantation position, are interconnected in a force-locking manner and/or form-locking manner.

6. Trial implant system in accordance with claim 5, **characterized in that** the at least one first and the at least one second latch element (52, 54) are arranged at the corresponding receptacles (36) and projections (82).

7. Trial implant system in accordance with any one of the preceding claims, **characterized in that** the connecting device (50; 150) has a rotationally symmetric configuration with respect to a trial implant longitudinal axis (32; 132).

8. Trial implant system in accordance with any one of the preceding claims, **characterized in that** the at least one first trial implant part (12; 112) forms a basic body (14; 114) of the trial implant system (10).

9. Trial implant system in accordance with any one of the preceding claims, **characterized in that** the at least one first trial implant part (12; 112) is of one-piece configuration.

10. Trial implant system in accordance with any one of the preceding claims, **characterized in that** the at least one second trial implant part (16; 116) is of one-piece configuration.

11. Trial implant system in accordance with any one of the preceding claims, **characterized by** at least two first trial implant parts (12; 112) of differing configuration.

12. Trial implant system in accordance with any one of the preceding claims, **characterized by** at least two second trial implant parts (16; 116) of differing configuration.

13. Trial implant system in accordance with any one of the preceding claims, **characterized in that** the at least one first trial implant part (12; 112) defines at least two trial implant part parameters (76, 78; 176, 178).

14. Trial implant system in accordance with claim 13, **characterized in that** the at least two first trial implant parts (12; 112) of differing configuration differ in at least one first trial implant part parameter (76, 176).

15. Trial implant system in accordance with any one of the preceding claims, **characterized in that** the at least one second trial implant part (16; 116) defines at least two trial implant part parameters (76, 78; 176, 178).

16. Trial implant system in accordance with claim 15, **characterized in that** the at least two second trial implant parts (16; 116) differ in at least one second trial implant part parameter (76, 78; 176, 178).

17. Trial implant system in accordance with any one of claims, **characterized by** at least one trial implant (18) in the form of a trial joint head (20) comprising a joint head part (22) and a connecting part (24).

18. Trial implant system in accordance with claim 17, **characterized in that** the connecting part (24) comprises a neck receptacle (26) which is capable of having inserted therein a pin-shaped neck section (28) of a joint stem (30) for connecting the trial joint head (20) to the joint stem (30).

19. Trial implant system in accordance with any one of the preceding claims, **characterized in that** the at least one second trial implant part (16) is configured in the form of a joint head cup (42).

## Revendications

1. Système d'implant d'essai modulaire (10) comprenant au moins un implant d'essai (18; 118), qui comporte au moins une première et au moins une deuxième pièce d'implant d'essai (16; 116) pouvant être reliée à ladite au moins une première pièce d'implant d'essai (12 ; 112), système
dans lequel il est prévu au moins un dispositif de liaison (50; 150) pour relier ladite au moins une première et ladite au moins une deuxième pièce d'implant d'essai (12, 16; 112, 116) dans une position d'implantation d'essai,
dans lequel ledit au moins un dispositif de liaison (50; 150) comprend au moins un premier et au moins un deuxième élément de liaison (52, 54; 152, 154),
dans lequel ledit un élément de liaison (52; 152) est agencé sur ladite au moins une première pièce d'implant d'essai (12 ; 112), et ledit autre élément de liaison (54; 154) est agencé sur ladite au moins une deuxième pièce d'implant d'essai (16; 116), et
dans lequel ledit au moins un premier et ledit au moins un deuxième élément de liaison (52, 54; 152, 154) sont de configuration mutuellement correspondante et sont en prise réciproque dans la position d'implantation d'essai,
**caractérisé en ce que** ledit au moins un dispositif de liaison (50; 150) est réalisé sous la forme d'une liaison par encliquetage.

2. Système d'implant d'essai selon la revendication 1, **caractérisé en ce que** ladite au moins une première et ladite au moins une deuxième pièce d'implant d'essai (12, 16; 112, 116) peuvent être reliées de manière amovible.

3. Système d'implant d'essai selon la revendication 1 ou la revendication 2, **caractérisé en ce que** ledit au moins un premier et/ou ledit au moins un deuxième élément de liaison (52, 54; 152, 154) sont réalisés sous la forme d'éléments d'encliquetage (58, 66; 158, 166) mutuellement correspondants.

4. Système d'implant d'essai selon la revendication 3, **caractérisé en ce que** ledit un élément d'encliquetage (58; 158) est réalisé sous la forme d'une rainure annulaire (58; 158) ou d'un secteur de rainure annulaire, et **en ce que** l'autre élément d'encliquetage correspondant (66; 166) est réalisé sous la forme d'un bourrelet annulaire (66 ; 166) ou d'un secteur de bourrelet annulaire, ce bourrelet annulaire (66 ; 166) ou secteur de bourrelet annulaire s'engageant ou venant en prise dans la rainure annulaire (58; 158) ou le secteur de rainure annulaire, en se trouvant dans la position d'implantation d'essai.

5. Système d'implant d'essai selon l'une des revendications précédentes, **caractérisé en ce que** ledit au moins un premier et/ou ledit au moins un deuxième élément de liaison (52, 54; 152, 154) sont réalisés sous la forme de logements d'accueil (36; 58; 158) et de protubérances (66; 82; 166), qui sont reliés mutuellement dans la position d'implantation d'essai, par une liaison par adhérence et/ou par complémentarité de formes.

6. Système d'implant d'essai selon la revendication 5, **caractérisé en ce que** ledit au moins un premier et ledit au moins un deuxième élément d'encliquetage (52, 54) sont agencés au niveau des logements d'accueil (36) et des protubérances (82) correspondants.

7. Système d'implant d'essai selon l'une des revendications précédentes, **caractérisé en ce que** le dispositif de liaison (50; 150) est de configuration à symétrie de rotation par rapport à un axe longitudinal d'implant d'essai (32; 132).

8. Système d'implant d'essai selon l'une des revendications précédentes, **caractérisé en ce que** ladite au moins une première pièce d'implant d'essai (12; 112) forme un corps de base (14; 114) du système d'implant d'essai (10).

9. Système d'implant d'essai selon l'une des revendications précédentes, **caractérisé en ce que** ladite au moins une première pièce d'implant d'essai (12; 112) est réalisée d'un seul tenant.

10. Système d'implant d'essai selon l'une des revendications précédentes, **caractérisé en ce que** ladite au moins une deuxième pièce d'implant d'essai (16; 116) est réalisée d'un seul tenant.

11. Système d'implant d'essai selon l'une des revendications précédentes, **caractérisé par** au moins deux premières pièces d'implant d'essai (12 ; 112) de conformation différente.

12. Système d'implant d'essai selon l'une des revendications précédentes, **caractérisé par** au moins deux deuxièmes pièces d'implant d'essai (16 ; 116) de conformation différente.

13. Système d'implant d'essai selon l'une des revendications précédentes, **caractérisé en ce que** ladite au moins une première pièce d'implant d'essai (12; 112) définit au moins deux paramètres de pièce d'implant d'essai (76, 78; 176, 178).

14. Système d'implant d'essai selon la revendication 13, **caractérisé en ce que** lesdites au moins deux premières pièces d'implant d'essai (12; 112) de conformation différente se différencient dans au moins un premier paramètre de pièce d'implant d'essai (76; 176).

15. Système d'implant d'essai selon l'une des revendications précédentes, **caractérisé en ce que** ladite au moins une deuxième pièce d'implant d'essai (16; 116) définit au moins deux paramètres de pièce d'implant d'essai (76, 78; 176, 178).

16. Système d'implant d'essai selon la revendication 15, **caractérisé en ce que** lesdites au moins deux deuxièmes pièces d'implant d'essai (16; 116) de conformation différente se différencient dans au moins un deuxième paramètre de pièce d'implant d'essai (76, 78; 176, 178).

17. Système d'implant d'essai selon l'une des revendications précédentes, **caractérisé par** au moins un implant d'essai (18) sous la forme d'une tête d'articulation d'essai (20), qui comprend une partie de tête d'articulation (22) et une partie de liaison (24).

18. Système d'implant d'essai selon la revendication 17, **caractérisé en ce que** la partie de liaison (24) comporte un logement de col (26) dans lequel peut être introduit un tronçon de col (28) d'une tige de prothèse d'articulation (30), pour relier la tête d'articulation d'essai (20) à la tige de prothèse d'articulation (30).

19. Système d'implant d'essai selon l'une des revendications précédentes, **caractérisé en ce que** ladite au moins une deuxième pièce d'implant d'essai (16) est réalisée sous la forme d'une cupule de tête d' articulation (42).
